(19) Europäisches Patentamt
European Patent Office
Office européen des brevets

(11) EP 4 230 664 A1

(12) **EUROPEAN PATENT APPLICATION**
published in accordance with Art. 153(4) EPC

(43) Date of publication:
**23.08.2023 Bulletin 2023/34**

(21) Application number: **21880228.8**

(22) Date of filing: **15.10.2021**

(51) International Patent Classification (IPC):
***C08F 20/56*** *(2006.01)* ***C09J 4/02*** *(2006.01)*
***C09J 7/38*** *(2018.01)*

(52) Cooperative Patent Classification (CPC):
**C08F 20/56; C09J 4/00; C09J 7/38**

(86) International application number:
**PCT/JP2021/038313**

(87) International publication number:
**WO 2022/080493 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.10.2020 JP 2020174005**

(71) Applicant: **KJ Chemicals Corporation**
**Tokyo 103-0023 (JP)**

(72) Inventors:
- **YASUNAGA Atsushi**
  **Yatsushiro-shi Kumamoto 866-0081 (JP)**
- **NARAZAKI Yu**
  **Yatsushiro-shi Kumamoto 866-0081 (JP)**
- **MAIMAITIYIMING Yilixiati**
  **Yatsushiro-shi Kumamoto 866-0081 (JP)**

(74) Representative: **ABG Intellectual Property Law, S.L.**
**Avenida de Burgos, 16D**
**Edificio Euromor**
**28036 Madrid (ES)**

(54) **POLYMERIZABLE COMPOSITION, PRODUCT OF POLYMERIZATION OF SAME, AND MOLDED ARTICLE OBTAINED USING THESE**

(57) An object of the present invention is to provide: a polymerizable composition that is excellent in wettability to substrates of various materials having a wide range of polarities from low to high polarity, such as a substrate of organic material, a substrate of inorganic material, and a substrate of an organic-inorganic hybrid material, has high transparency and curability, and can be cured to provide a cured product having excellent water resistance; a product of polymerization of the polymerizable composition; and various molded articles obtained using the polymerizable composition and the product of polymerization of the polymerizable composition. A polymerizable composition containing N-substituted (meth)acrylamide (A) represented by a general formula [1].

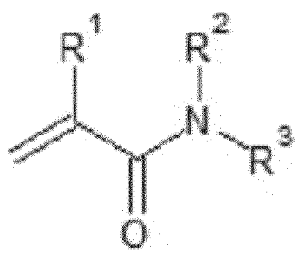

In the formula, $R^1$ represents a hydrogen atom or a methyl group, one of $R^2$ and $R^3$ represents a chain hydrocarbon group having 6 or more carbon atoms or a cyclic hydrocarbon group having 6 or more carbon atoms, the other represents a hydrogen atom, a chain hydrocarbon group having 1 or more carbon atoms, or a cyclic hydrocarbon group having 3 or more carbon atoms, and $R^2$ and $R^3$ include a group in which a 6- or more-membered saturated ring is formed together with a nitrogen atom carrying $R^2$ and $R^3$.

EP 4 230 664 A1

## Description

TECHNICAL FIELD

The present invention relates to a polymerizable composition, a product of polymerization of the polymerizable composition, and a molded article obtained using the polymerizable composition and the product of polymerization.

BACKGROUND ART

In recent years, N-substituted (meth)acrylamide has been widely applied as a raw material monomer for adhesives, adhesives for optical members, active energy ray-curable adhesives for polarizing plates, inks for inkjet, resin compositions for stereolithography, encapsulants for semiconductors and electronic materials, coating agents for glass and resin molded articles, and the like (PATENT LITERATURES 1 to 4). Specifically, it has been often reported that N-substituted (meth)acrylamide is used as a substitute for (meth)acrylic acid since it has high cohesive force of amide group, excellent adhesiveness to various substrates, and no corrosiveness to metals or metal oxides (PATENT LITERATURES 5 to 7). However, general-purpose N-substituted (meth)acrylamide contains many hydrophilic monomers soluble in water, and it has been pointed out that water resistance is insufficient depending on content thereof and use of the molded article to be obtained.

Further, N-substituted (meth)acrylamide is often used as a constituent component of an active energy ray-curable resin, but a method for improving water resistance has not been proposed yet when it is blended in a long-life product such as an electronic material or an optical material.

CITATION LIST

PATENT LITERATURE

PATENT LITERATURE 1: JP-A-2008-287207
PATENT LITERATURE 2: JP-A-2011-122013
PATENT LITERATURE 3: JP-A-2001-310918
PATENT LITERATURE 4: JP-A-2010-155889
PATENT LITERATURE 5: JP-A-2011-137181
PATENT LITERATURE 6: JP-A-2010-235646
PATENT LITERATURE 7: JP-A-2013-256552

SUMMARY OF THE INVENTION

PROBLEMS TO BE SOLVED BY THE INVENTION

A first object of the present invention is to provide: a polymerizable composition that is excellent in wettability to substrates of various materials having a wide range of polarities from low to high polarity, such as a substrate of organic material, a substrate of inorganic material, and a substrate of an organic-inorganic hybrid material, has high transparency and curability, and can be cured to provide a cured product having excellent water resistance; a product of polymerization of the polymerizable composition; and the like. A second object of the present invention is to provide: an adhesive composition containing the polymerizable composition and/or the product of polymerization of the polymerizable composition, and the like, having adhesiveness and adhesive strength to various substrates, and having high transparency, water resistance, stain resistance, yellowing resistance, and durability; and a multi-layer laminate product of an adhesive layer containing the adhesive composition and various substrates. Further, a third object of the present invention is to provide: a glue composition for bonding the same or different types of materials, containing the polymerizable composition and/or the product of polymerization of the polymerizable composition, and having high bond strength to various substrates, high impact resistance, and water resistance; a coating agent composition having high wettability and adhesiveness to various substrates and exhibiting high surface hardness and water resistance by being cured; a hair cosmetic having moisture resistance, smoothness, anti-stickiness, good texture, and stability over time; an oil-in-water emulsified cosmetic composition that does not exhibit skin irritation, and has excellent emulsification stability, usability, and stability over time; an ink having high adhesiveness to various substrates, excellent in printing properties such as pigment dispersibility, surface dryness, ejection stability, and sharpness, and having high curability, yellowing resistance, and water resistance; and a three-dimensional modeling ink composition that can accurately form a three-dimensional shaped article having high strength, heat resistance, and water resistance, and has excellent curing shrinkage resistance.

SOLUTIONS TO THE PROBLEMS

As a result of intensive studies in view of the above, the present inventors have found that a polymerizable composition containing an amphiphilic N-substituted (meth)acrylamide having a specific structure, which has at least one chain or cyclic hydrocarbon group having 6 or more carbon atoms as a hydrophobic group and a (meth)acrylamide group as a hydrophilic group, can solve the above problems, and have completed the present invention.

That is, the present invention provides the following:

(1) A polymerizable composition containing N-substituted (meth)acrylamide (A) represented by a general formula [1].

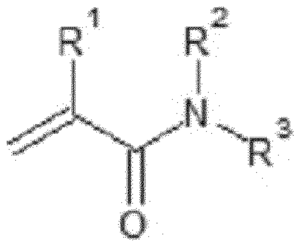

General Formula [1]

In the formula, $R^1$ represents a hydrogen atom or a methyl group, one of $R^2$ and $R^3$ represents a chain hydrocarbon group having 6 or more carbon atoms or a cyclic hydrocarbon group having 6 or more carbon atoms, the other represents a hydrogen atom, a chain hydrocarbon group having 1 or more carbon atoms, or a cyclic hydrocarbon group having 3 or more carbon atoms, and $R^2$ and $R^3$ include a group in which a 6- or more-membered saturated ring is formed together with a nitrogen atom carrying $R^2$ and $R^3$.

(2) The polymerizable composition according to the above (1), in which the N-substituted (meth)acrylamide (A) is N-mono-substituted (meth)acrylamide and N,N-disubstituted (meth)acrylamide, and has as substituents one or more structures selected from chain saturated and unsaturated structures and cyclic saturated and unsaturated structures having 6 or more and 36 or less carbon atoms.

(3) The polymerizable composition according to the above (1) or (2), in which a saturated water absorption of a cured product is 10% or less.

(4) The polymerizable composition according to any one of the above (1) to (3), in which the N-substituted (meth)acrylamide (A) has a surface tension of 24.0 to 46.0 $mN \cdot m^{-1}$.

(5) The polymerizable composition according to any one of the above (1) to (4), in which a content of the N-substituted (meth)acrylamide (A) is 1 wt% or more based on the entire polymerizable composition.

(6) A product of polymerization obtained by polymerizing the polymerizable composition according to any one of the above (1) to (5) with active energy rays and/or heat.

(7) The polymerizable composition according to any one of the above (1) to (5), further containing one or more selected from a polymerization initiator, a compound having an unsaturated bond (excluding N-substituted (meth)acrylamide (A) and a product of polymerization using N-substituted (meth)acrylamide (A)), a non-polymerizable oligomer and a non-polymerizable polymer (excluding the product of polymerization using N-substituted (meth)acrylamide (A)), and the product of polymerization according to claim 6.

(8) An adhesive composition containing the polymerizable composition according to any one of the above (1) to (5) and (7) or the product of polymerization according to the above (6), or the polymerizable composition according to any one of the above (1) to (5) and (7) or the product of polymerization according to the above (6) and a crosslinking agent.

(9) A multi-layer laminate product including an adhesive layer containing the adhesive composition according to the above (8) and an organic and/or inorganic substrate, in which the organic and/or inorganic substrate has a surface tension of 22.6 to 59.0 $mN \cdot m^{-1}$.

(10) A glue composition containing the polymerizable composition according to any one of the above (1) to (5) and (7) or the product of polymerization according to the above (6) and a crosslinking agent.

(11) A glue composition containing the polymerizable composition according to any one of the above (1) to (5) and (7) or the product of polymerization according to the above (6) and a crosslinking agent, in which an absolute value

of a difference in surface tension between two kinds of different types of adherends is 37.0 $mN \cdot m^{-1}$ or less.

(12) A cosmetic composition containing the polymerizable composition according to any one of the above (1) to (5) and (7) or the product of polymerization according to the above (6).

(13) A coating agent composition containing the polymerizable composition according to any one of the above (1) to (5) and (7) or the product of polymerization according to the above (6), or the polymerizable composition according to any one of the above (1) to (5) and (7) or the product of polymerization according to the above (6) and a crosslinking agent.

(14) An ink composition containing the polymerizable composition according to any one of the above (1) to (5) and (7) or the product of polymerization according to the above (6), or the polymerizable composition according to any one of the above (1) to (5) and (7) or the product of polymerization according to the above (6) and a crosslinking agent.

(15) An ink composition for use in three-dimensional modeling, containing the polymerizable composition according to any one of the above (1) to (5) and (7) or the product of polymerization according to the above (6), or the polymerizable composition according to any one of the above (1) to (5) and (7) or the product of polymerization according to the above (6) and a crosslinking agent.

## EFFECTS OF THE INVENTION

According to the present invention, it is possible to obtain: a polymerizable composition containing N-substituted (meth)acrylamide (A) having a specific structure, which has high transparency and good curability, and is excellent in wettability to substrates of various materials having a wide range of polarities from low to high polarity, such as a substrate of organic material, a substrate of inorganic material, and a substrate of an organic-inorganic hybrid material, due to well-balanced amphiphilicity of the N-substituted (meth)acrylamide (A); a product of polymerization of the polymerizable composition; and the like. It is possible to provide: an adhesive composition that exhibits adhesiveness and adhesive strength to various substrates, and has high transparency, stain resistance, yellowing resistance, and durability, by containing the obtained polymerizable composition and/or the product of polymerization of the polymerizable composition, and the like; and a multi-layer laminate product of an adhesive layer containing the adhesive composition and various substrates. Further, the present invention can provide: a glue composition for the same or different types of materials having high bond strength to various substrates, high impact resistance, and water resistance, by containing the polymerizable composition and/or the product of polymerization of the polymerizable composition; a hair cosmetic having moisture resistance, smoothness, anti-stickiness, good texture, and stability over time; an oil-in-water emulsified cosmetic composition that does not exhibit skin irritation, and has excellent emulsification stability, usability, and stability over time; a coating agent having high wettability and adhesiveness to various substrates and exhibiting high surface hardness and water resistance by being cured; an ink having high adhesiveness to various substrates, excellent in printing properties such as pigment dispersibility, surface dryness, ejection stability, and sharpness, and having high curability and yellowing resistance; and a three-dimensional modeling ink composition that can accurately form a three-dimensional shaped article having high strength, heat resistance, and water resistance, and has excellent curing shrinkage resistance.

## DESCRIPTION OF THE EMBODIMENTS

A first embodiment of the present invention is a polymerizable composition. A second embodiment is a product of polymerization obtained by polymerizing the polymerizable composition of the first embodiment with active energy rays and/or heat. A third embodiment is the polymerizable composition of the first embodiment, further containing one or more selected from a polymerization initiator, a compound having an unsaturated bond (excluding N-substituted (meth)acrylamide (A) and a product of polymerization using N-substituted (meth)acrylamide (A)), a non-polymerizable oligomer and a non-polymerizable polymer (excluding a product of polymerization using N-substituted (meth)acrylamide (A)), and the product of polymerization of the second embodiment. A fourth embodiment is the product of polymerization of the second embodiment, further containing one or more selected from a polymerization initiator, a compound having an unsaturated bond (excluding N-substituted (meth)acrylamide (A) and a product of polymerization using N-substituted (meth)acrylamide (A)), a non-polymerizable oligomer and a non-polymerizable polymer (excluding a product of polymerization using N-substituted (meth)acrylamide (A)), and a crosslinking agent having two or more reactive functional groups in a molecule (excluding a compound having two or more unsaturated bonds in a molecule). Hereinafter, the first to fourth embodiments of the present invention will be collectively described.

The polymerizable composition according to the first embodiment of the present invention contains N-substituted (meth)acrylamide (A) represented by the following general formula [1].

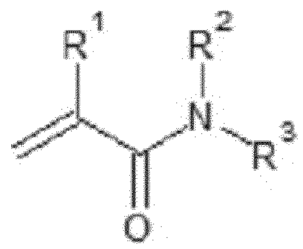

General Formula [1]

In the general formula [1], $R^1$ represents a hydrogen atom or a methyl group, one of $R^2$ and $R^3$ represents a chain hydrocarbon group having 6 or more carbon atoms or a cyclic hydrocarbon group having 6 or more carbon atoms, the other represents a hydrogen atom, a chain hydrocarbon group having 1 or more carbon atoms, or a cyclic hydrocarbon group having 3 or more carbon atoms, and $R^2$ and $R^3$ include a group in which a 6- or more-membered saturated ring is formed together with a nitrogen atom carrying $R^2$ and $R^3$. The 6- or more-membered saturated ring may or may not contain a hetero atom. Further, $R^2$ and $R^3$ may or may not contain a heteroatom-containing substituent, and when contained, the number of heteroatoms is 3 or less. The heteroatom refers to an oxygen atom, a sulfur atom, a nitrogen atom, or a boron atom. Note that N-substituted (meth)acrylamide not included in the general formula [1] is also referred to as N-substituted (meth)acrylamide (B).

The product of polymerization according to the second embodiment of the present invention is a product of polymerization obtained by polymerizing the polymerizable composition of the first embodiment with active energy rays and/or heat. The product of polymerization is a soluble polymer having no crosslinked structure, may be a homopolymer of any one kind of monomer selected from the N-substituted (meth)acrylamide (A), may be a copolymer of A obtained by copolymerizing two or more kinds of monomers optionally selected from A in any proportion, or may be a copolymer obtained by copolymerizing one or more monomers optionally selected from A with a copolymerizable monomer other than A in any proportion. Note that in the copolymer obtained from the N-substituted (meth)acrylamide (A) and the monomer other than A, the content of A in the copolymer is preferably 1 wt% or more based on a total weight of the copolymer.

The N-substituted (meth)acrylamide (A) used in the first to fourth embodiments of the present invention (hereinafter collectively referred to as the present embodiment) is amphiphilic, and has at least one substituent selected from the group consisting of a chain hydrocarbon group having 6 or more carbon atoms, a cyclic hydrocarbon group having 6 or more carbon atoms, and a 6- or more-membered saturated ring containing a nitrogen atom carrying $R^2$ and $R^3$, as a hydrophobic substituent capable of imparting wettability to a low polarity substrate in the molecule, and a hydrophilic (meth)acrylamide group capable of imparting wettability to a high-polaritysubstrate. The polymerizable composition according to the present embodiment contains the N-substituted (meth)acrylamide (A), so that compatibility of components constituting the composition is good, high transparency can be obtained, and wettability to substrates of various materials having a wide range of polarities from low to high polarity, such as a substrate of organic material, a substrate of inorganic material, and a substrate of an organic-inorganic hybrid material is good. Furthermore, since the N-substituted (meth)acrylamide (A) exhibits sufficient curability and polymerizability to active energy rays and/or heat, the polymerizable composition containing the N-substituted (meth)acrylamide (A) has high curability and polymerizability. Due to a synergistic effect of good wettability to various substrates having a wide range of polarities and strong cohesive force between amide groups, an adhesive composition and a glue composition molded from the polymerizable composition containing the N-substituted (meth)acrylamide (A) and/or the product of polymerization obtained by polymerizing the polymerizable composition (hereinafter also referred to as the product of polymerization of the polymerizable composition or the product of polymerization) are excellent in adhesiveness to various substrates. Therefore, as a molded article of the polymerizable composition and/or the product of polymerization of the polymerizable composition, the adhesive composition having high adhesive strength and stain resistance (reworkability) and the glue composition having high bond strength, impact resistance, and water resistance can be obtained. The polymerizable composition and/or the product of polymerization of the polymerizable composition according to the present embodiment can be applied not only to the adhesive composition and the glue composition but also to various uses such as a coating agent composition, a cosmetic composition, an ink composition, an inkjet ink composition, and an ink composition used for three-dimensional modeling, by utilizing characteristics of the N-substituted (meth)acrylamide (A) described above.

From the viewpoint of improving the wettability of the polymerizable composition according to the present embodiment to the low polarity substrate, the number of carbon atoms of the chain hydrocarbon group or the cyclic hydrocarbon group in $R^2$ and $R^3$ in the general formula [1] is preferably 8 or more, more preferably 12 or more, and still more preferably 16 or more. On the other hand, the number of carbon atoms of the chain hydrocarbon group or the

cyclic hydrocarbon group is not particularly limited, but is usually 36 or less, preferably 24 or less, and more preferably 22 or less, in order to sufficiently maintain the wettability of the polymerizable composition to a high polaritysubstrate, that is, from the viewpoint of balance of wettability to the low polarity substrate and the high polarity substrate. In addition, the number of members of the saturated ring containing the nitrogen atom carrying $R^2$ and $R^3$ in the general formula [1] is preferably 6 or more and 12 or less, from the viewpoint of balancing the wettability of the polymerizable composition to the low polarity substrate and the high polarity substrate.

The N-substituted (meth)acrylamide (A) used in the present embodiment is N-mono-substituted (meth)acrylamide and N,N-disubstituted (meth)acrylamide, and preferably has, as substituents, one or more structures selected from chain saturated and unsaturated structures and cyclic saturated and unsaturated structures having 6 or more and 36 or less carbon atoms, as described above. In addition, the N-substituted (meth)acrylamide (A) more preferably has at least one substituent having an unsaturated structure. Generally, in aliphatic compounds having the same number of carbon atoms, those containing an unsaturated bond in their structure tend to have a lower melting point than those not containing an unsaturated bond. As described later, when the N-substituted (meth)acrylamide (A) is a liquid at normal temperature, the content of the N-substituted (meth)acrylamide (A) in the polymerizable composition of the present embodiment can be arbitrarily adjusted to some extent. Therefore, by containing one or more substituents having an unsaturated structure, the N-substituted (meth)acrylamide (A) has an effect of lowering the melting point of the N-substituted (meth)acrylamide (A), which is more preferred.

In the polymerizable composition according to the present embodiment, a saturated water absorption of a cured product at normal temperature is preferably 10% or less. In the present specification, the normal temperature indicates 5°C to 35°C under atmospheric pressure. The saturated water absorption of the cured product is a water absorption in a saturated water absorption state, and can be calculated by a method described later. In addition, it is sufficient that the saturated water absorption state is reached, and a method, conditions, and the like for reaching that state are not limited. When the saturated water absorption of the cured product is 10% or less, the cured product has sufficient water resistance. Since the lower the saturated water absorption, the higher the water resistance of the cured product, the saturated water absorption of the cured product is more preferably 7% or less, and particularly preferably 5% or less.

A surface tension of the N-substituted (meth)acrylamide (A) is preferably 24.0 to 46.0 $mN \cdot m^{-1}$. In the present specification, the surface tension of the N-substituted (meth)acrylamide (A) is a calculated value at 23°C obtained by the Meissner method (Chemical Engineering Handbook Revision 7, page 66), and an average error thereof is 3%.

When the surface tension of the N-substituted (meth)acrylamide (A) is within the above numerical range, and the polymerizable composition containing the N-substituted (meth)acrylamide (A) is applied to a substrate having a different polarity, molecules can be arranged in accordance with the polarity of the substrate, and as a result, good wettability to various substrates exhibiting a wide range of polarities described above is exhibited, and adhesiveness of the adhesive composition, the glue composition, or the like which is the molded article of the polymerizable composition and/or the product of polymerization of the polymerizable composition is also improved. From these viewpoints, the surface tension of the N-substituted (meth)acrylamide (A) is more preferably 28.0 to 36.0 $mN \cdot m^{-1}$, and particularly preferably 30.0 to 33.0 $mN \cdot m^{-1}$.

The N-substituted (meth)acrylamide (A) is preferably a liquid at normal temperature. Since the N-substituted (meth)acrylamide (A) is a liquid at normal temperature, the content of the N-substituted (meth)acrylamide (A) in the polymerizable composition according to the present embodiment can be arbitrarily adjusted to some extent without performing operations such as heating, and the transparency of the obtained polymerizable composition is easily maintained. The N-substituted (meth)acrylamide (A) is more preferably a liquid at 30°C or lower, and still more preferably a liquid at 25°C or lower. As used herein, the term "liquid" includes a liquid state having fluidity and a wax state having no fluidity.

In the polymerizable composition according to the present embodiment, the content of the N-substituted (meth)acrylamide (A) may be 1 wt% or more and 100 wt% based on a total weight of the polymerizable composition, and the content of A can be appropriately adjusted according to a specific use of the polymerizable composition. If the polymerizable composition contains 1 wt% of the N-substituted (meth)acrylamide (A), an effect of improving curability and polymerizability to active energy rays and/or heat, transparency, and wettability to various substrates exhibiting a wide range of polarities of the polymerizable composition can be obtained, and in order to obtain a better balance of such curability, polymerizability, transparency, and wettability, the content of the N-substituted (meth)acrylamide (A) is more preferably 5 to 90 wt%, even more preferably 10 to 80 wt%, and particularly preferably 20 to 70 wt%.

When the N-substituted (meth)acrylamide (A) has, as a substituent, an aliphatic hydrocarbon group not containing an unsaturated bond and having 6 or more carbon atoms, the aliphatic hydrocarbon group may have a linear, branched, or cyclic structure. Examples of the N-substituted (meth)acrylamide include n-hexyl (meth)acrylamide, sec-hexyl (meth)acrylamide, tert-hexyl (meth)acrylamide, n-heptyl (meth)acrylamide, sec-heptyl (meth)acrylamide, tert-heptyl (meth)acrylamide, n-octyl (meth)acrylamide, sec-octyl (meth)acrylamide, tert-octyl (meth)acrylamide, 2-ethylhexyl (meth)acrylamide, N,N-di(2-ethylhexyl)acrylamide, n-nonyl (meth)acrylamide, n-decyl (meth)acrylamide, n-undecyl (meth)acrylamide, n-dodecyl (meth)acrylamide, n-tridecyl (meth)acrylamide, n-trimethyldecyl (meth)acrylamide, n-tetradecyl (meth)acrylamide, n-hexadecyl (meth)acrylamide, stearyl (meth)acrylamide, n-eicosyl (meth)acrylamide, n-docosyl (meth)acrylamide, n-tetracosyl (meth)acrylamide, N-cyclohexyl (meth)acrylamide, N,N-dicyclohexyl (meth)acrylamide, N-cyclohexyl-N-methyl (meth)acrylamide, N-cyclohexyl-N-ethyl (meth)acrylamide, N-cyclohexyl-N-propyl (meth)acrylamide, N-cyclohexyl-N-butyl (meth)acrylamide, N-cyclohexyl-N-pentyl (meth)acrylamide, N-cyclohexyl-N-hexyl (meth)acrylamide, N-phenyl (meth)acrylamide, N-(meth)acryloylpiperidine, N-(meth)acryloyl-2-methylpiperidine, N-(meth)acryloyl-3-methylpiperidine, N-(meth)acryloyl-4-methylpiperidine, N-(meth)acryloyl-2,6-dimethylpiperidine, N-(meth)acryloyl-3,5-dimethylpiperidine, N-(meth)acryloyl-3,3-dimethylpiperidine, N-(meth)acryloyl-4,4-dimethylpiperidine, N-(meth)acryloyl-2,2,6,6-tetramethylpiperidine, N-(meth)acryloyl-2-methyl-5-ethylpiperidine, N-(meth)acryloyl-4-methyl-4-ethylpiperidine, N-(meth)acryloyl-2-ethylpiperidine, N-(meth)acryloyl-3-ethylpiperidine, N-(meth)acryloyl-4-ethylpiperidine, N-(meth)acryloyl-2-propylpiperidine, N-(meth)acryloyl-3-propylpiperidine, N-(meth)acryloyl-4-propylpiperidine, N-(meth)acryloyl-3-isopropylpiperidine, N-(meth)acryloyl-4-isopropylpiperidine, N-(meth)acryloylhexamethyleneimine, N-(meth)acryloyl-2-methylhexamethyleneimine, N-(meth)acryloyl-3-methylhexamethyleneimine, N-(meth)acryloyl-4-methylhexamethyleneimine, N-(meth)acryloyl-2-ethylhexamethyleneimine, N-(meth)acryloyl-3-ethylhexamethyleneimine, N-(meth)acryloyl-4-ethylhexamethyleneimine, N-(meth)acryloyl-3-propylhexamethyleneimine, N-(meth)acryloyl-4-propylhexamethyleneimine, N-(meth)acryloyl-3-propylhexamethyleneimine, N-(meth)acryloyl-4-isopropylhexamethyleneimine, N-(meth)acryloyl-3,5-isopropylhexamethyleneimine, N-(meth)acryloyl-4,4-dimethylhexamethyleneimine, N-(meth)acryloylheptamethyleneimine, N-(meth)acryloyloctamethyleneimine, N-(meth)acryloyldecamethyleneimine, dopamine (meth)acrylamide, and 3-(meth)acrylamidophenylboronic acid. Among these, for example, from the viewpoint of easily obtaining industrial products, n-hexyl (meth)acrylamide, n-octyl (meth)acrylamide, tert-octyl (meth)acrylamide, 2-ethylhexyl (meth)acrylamide, N,N-di(2-ethylhexyl)acrylamide, n-nonyl (meth)acrylamide, n-decyl (meth)acrylamide, n-dodecyl (meth)acrylamide, n-tridecyl (meth)acrylamide, n-trimethyldecyl (meth)acrylamide, n-tetradecyl (meth)acrylamide, n-hexadecyl (meth)acrylamide, stearyl (meth)acrylamide, N-cyclohexyl (meth)acrylamide, N,N-dicyclohexyl (meth)acrylamide, N-cyclohexyl-N-methyl (meth)acrylamide, N-(meth)acryloylpiperidine, N-(meth)acryloyl-2-methylpiperidine, N-(meth)acryloyl-4-methylpiperidine, N-(meth)acryloyl-2,6-dimethylpiperidine, N-(meth)acryloyl-3,5-dimethylpiperidine, N-phenyl (meth)acrylamide, dopamine (meth)acrylamide, and 3-(meth)acrylamidophenyl boronic acid are preferred.

When the N-substituted (meth)acrylamide (A) has, as a substituent, an aliphatic hydrocarbon group containing an unsaturated bond and having 6 or more carbon atoms, the aliphatic hydrocarbon group may have a linear, branched, or cyclic structure. Examples of the N-substituted (meth)acrylamide include hexenyl (meth)acrylamide, heptenyl (meth)acrylamide, octenyl (meth)acrylamide, nonenyl (meth)acrylamide, decenyl (meth)acrylamide, undecenyl (meth)acrylamide, dodecenyl (meth)acrylamide, tetradecenyl (meth)acrylamide, hexadecenyl (meth)acrylamide, oleyl (meth)acrylamide, icosenyl (meth)acrylamide, docosenyl (meth)acrylamide, tetracosenyl (meth)acrylamide, octadecadienyl (meth)acrylamide, icosadienyl (meth)acrylamide, docosadienyl (meth)acrylamide, tetracosadienyl (meth)acrylamide, octadecatrienyl (meth)acrylamide, icosatrienyl (meth)acrylamide, docosatrienyl (meth)acrylamide, tetracosatrienyl (meth)acrylamide, octadecatetraenyl (meth)acrylamide, icosatetraenyl (meth)acrylamide, docosatetraenyl (meth)acrylamide, tetracosatetraenyl (meth)acrylamide, octadecapentaenyl (meth)acrylamide, icosapentaenyl (meth)acrylamide, docosapentaenyl (meth)acrylamide, tetracosapentaenyl (meth)acrylamide, docosahexaenyl (meth)acrylamide, and tetracosahexaenyl (meth)acrylamide. Among these, for example, from the viewpoint of easily obtaining industrial products, octenyl (meth)acrylamide, nonenyl (meth)acrylamide, undecenyl (meth)acrylamide, and oleyl (meth)acrylamide are preferred.

The polymerizable composition according to the present embodiment may further contain, in addition to N-substituted (meth)acrylamide (A), one or more selected from a polymerization initiator, a compound having an unsaturated bond (excluding N-substituted (meth)acrylamide (A) and a product of polymerization using N-substituted (meth)acrylamide (A)), a non-polymerizable oligomer and a non-polymerizable polymer (excluding a product of polymerization using N-substituted (meth)acrylamide (A)), and the product of polymerization of the polymerizable composition. When the polymerizable composition further contains a polymerization initiator, curability and polymerizability to active energy rays and/or heat are further improved, and when the polymerizable composition further contains the compound having an unsaturated bond, the polymerizable composition can be more suitably used as the adhesive composition, the glue composition, the coating agent composition, and various ink compositions. In addition, when the polymerizable composition further contains the non-polymerizable oligomer and/or the non-polymerizable polymer, a viscosity of the polymerizable composition and flexibility of the cured product of the polymerizable composition are easily adjusted. In particular, when the polymerizable composition further contains the product of polymerization of the polymerizable composition, the adhesive strength of the adhesive composition which is the molded article of the polymerizable composition or the product of polymerization of the polymerizable composition to various substrates and the bond strength of the glue composition to various substrates are improved. Hereinafter, the polymerizable composition represents both a polymerizable composition not containing the product of polymerization of the polymerizable composition and a polymerizable composition containing the product of polymerization of the polymerizable composition.

Examples of the compound having an unsaturated bond include a monofunctional monomer having one unsaturated bond, a monofunctional oligomer, a polyfunctional monomer or polyfunctional oligomer having two or more unsaturated bonds, and a polymerizable polymer having an unsaturated bond. These compounds having an unsaturated bond are not limited due to the specific use of the polymerizable composition, but the monofunctional monomer is used, for example, for the purpose of adjusting the viscosity of the polymerizable composition to a low viscosity side, the polymerizable polymer is used, for example, for the purpose of adjusting the viscosity of the polymerizable composition to a high viscosity side, and the polyfunctional monomer, the polyfunctional oligomer, and the polymerizable polymer are used, for example, for the purpose of adjusting the curability of the polymerizable composition and a cross-linking rate of the resulting cured product. The components other than the N-substituted (meth)acrylamide (A) contained in the polymerizable composition of the present embodiment may be added alone or in combination of two or more thereof. Each content may be appropriately adjusted according to the specific use, but it is preferred that the content of the monofunctional monomer is 1 to 99 wt%, the content of the polyfunctional monomer is 0.05 to 50 wt%, the content of the polyfunctional oligomer is 0.05 to 50 wt%, the content of the polymerizable or non-polymerizable polymer is 0.01 to 10 wt%, and the content of the product of polymerization of the polymerizable composition is 0.01 to 10 wt% based on the total weight of the polymerizable composition. In the present specification, homopolymers or copolymers of various monomers having a weight average molecular weight (Mw) of 1,000 or more and less than 10,000 are classified as oligomers, and those having an Mw of 10,000 or more are classified as polymers.

The polyfunctional monomer is a monomer having two or more unsaturated bonds selected from a (meth)acrylate group, a (meth)acrylamide group, a vinyl group, an allyl group, and a maleimide group in the molecule, and examples thereof include polyfunctional (meth)acrylate and polyfunctional (meth)acrylamide. Generally, a polyfunctional monomer having 10 or less unsaturated bonds in the molecule is suitably used.

Examples of the polyfunctional (meth)acrylate include ethylene glycol di(meth)acrylate, diethylene glycol di(meth)acrylate, propylene glycol di(meth)acrylate, dipropylene glycol di(meth)acrylate, tripropylene glycol di(meth)acrylate, tetraethylene glycol di(meth)acrylate, ditetraethylene glycol di(meth)acrylate, polyethylene glycol di(meth)acrylate, polypropylene glycol di(meth)acrylate, polytetramethylene glycol di(meth)acrylate, butylene glycol di(meth)acrylate, 1,3-butanediol di(meth)acrylate, 1,4-butanediol di(meth)acrylate, neopentyl glycol di(meth)acrylate, 1,6-hexanediol di(meth)acrylate, 1,6-hexanediol ethylene oxide-modified di(meth)acrylate, 1,7-heptanediol di(meth)acrylate, 1,8-octanediol di(meth)acrylate, 1,9-nonanediol di(meth)acrylate, hydroxypivalic acid neopentyl glycol di(meth)acrylate, dicyclopentanyl di(meth)acrylate, caprolactone-modified dicyclopentenyl di(meth)acrylate, ethylene oxide-modified phosphoric acid di(meth)acrylate, glycerin di(meth)acrylate, pentaerythritol tetra(meth)acrylate, pentaerythritol tri(meth)acrylate, pentaerythritol di(meth)acrylate, dipentaerythritol tri(meth)acrylate, dipentaerythritol hexa(meth)acrylate, dipentaerythritol penta(meth)acrylate, dipentaerythritol tetra(meth)acrylate, ethylene glycol diglycidyl ether di(meth)acrylate, diethylene glycol diglycidyl ether (meth)acrylate, diglycidyl phthalate di(meth)acrylate, glycerin polyglycidyl ether poly(meth)acrylate, trimethylolethane tri(meth)acrylate, trimethylolpropane tri(meth)acrylate, tricyclodecane dimethanol di(meth)acrylate, ethylene oxide-modified bisphenol A type di(meth)acrylate, propylene oxide-modified bisphenol A type di(meth)acrylate, cyclohexanedimethanol di(meth)acrylate, acrylate ester (dioxane glycol diacrylate), alkoxylated hexanediol di(meth)acrylate, alkoxylated cyclohexanedimethanol di(meth)acrylate, epoxy (meth)acrylate, urethane (meth)acrylate, isocyanuric acid ethylene oxide-modified diacrylate, isocyanuric acid ethylene oxide-modified tri(meth)acrylate, tri(meth)acryloyloxyethoxytrimethylolpropane, ethylene oxide-modified dipentaerythritol penta(meth)acrylate, ethylene oxide-modified dipentaerythritol hexa(meth)acrylate, ethylene oxide-modified pentaerythritol tri(meth)acrylate, ethylene oxide-modified pentaerythritol tetra(meth)acrylate, and succinic acid-modified pentaerythritol tri(meth)acrylate.

Examples of the polyfunctional (meth)acrylamide include methylenebis(meth)acrylamide, ethylenebis(meth)acrylamide, diallyl (meth)acrylamide, N-[tris(3-(meth)acrylamidopropoxymethyl)methyl] (meth)acrylamide, N,N-bis(2-(meth)acrylamidoethyl) (meth)acrylamide, 4,7,10-trioxa-1,13-tridecanebis(meth)acrylamide, and N,N'-1,2-ethanediylbis[N-(2-(meth)acrylamidoethyl)] (meth)acrylamide. These polyfunctional monomers may be used alone or in combination of two or more thereof.

Examples of the monofunctional monomer include radical polymerizable compounds having a reactive double bond in the molecule, such as monofunctional (meth)acrylate, monofunctional (meth)acrylamide, styrene, an alkoxy group-containing monomer, a vinyl group-containing monomer, an allyl group-containing monomer, and a maleimide group-containing monomer.

Examples of the monofunctional (meth)acrylate include methyl (meth)acrylate, ethyl (meth)acrylate, propyl (meth)acrylate, isopropyl (meth)acrylate, n-butyl (meth)acrylate, isobutyl (meth)acrylate, hydroxyethyl (meth)acrylate, tert-butyl (meth)acrylate, 2-ethylhexyl (meth)acrylate, isodecyl (meth)acrylate, lauryl (meth)acrylate, stearyl (meth)acrylate, isostearyl (meth)acrylate, tridecyl (meth)acrylate, methoxyethyl (meth)acrylate, ethoxyethyl (meth)acrylate, propoxyethyl (meth)acrylate, butoxyethyl (meth)acrylate, methoxydiethylene glycol (meth)acrylate, methoxytriethylene glycol (meth)acrylate, methoxytetraethylene glycol (meth)acrylate, 2-(2-ethoxyethoxy) ethyl acrylate, phenoxyethyl (meth) acrylate, phenoxydiethylene glycol (meth)acrylate, phenoxytetraethylene glycol (meth)acrylate, phenoxyhexaethylene glycol (meth)acrylate, methoxydipropylene glycol (meth)acrylate, methoxytripropylene glycol (meth)acrylate, cyclohexyl

(meth)acrylate, tert-butylcyclohexyl (meth)acrylate, benzyl (meth)acrylate, dicyclopentanyl (meth)acrylate, dicyclopentenyl (meth)acrylate, bornyl (meth)acrylate, isobornyl (meth)acrylate, tetrahydrofuryl acrylate, 2-methyl-2-adamantyl (meth)acrylate, allyl (meth)acrylate, hydroxyethyl (meth)acrylate, hydroxypropyl (meth)acrylate, 2-hydroxybutyl (meth)acrylate, 4-hydroxybutyl (meth)acrylate, and hydroxyhexyl (meth)acrylate.

Examples of the monofunctional (meth)acrylamide include N-methyl (meth)acrylamide, N-ethyl (meth)acrylamide, N-isopropyl (meth)acrylamide, N-butyl (meth)acrylamide, N-methoxymethyl (meth)acrylamide, N-ethoxymethyl (meth)acrylamide, N-methoxyethyl (meth)acrylamide, N-ethoxyethyl (meth)acrylamide, N-n-butoxymethyl (meth)acrylamide, N-isobutoxymethyl (meth)acrylamide, N-(2-hydroxyethyl) acrylamide, N-[3-(dimethylamino)] propylacrylamide, N,N-dimethyl (meth)acrylamide, N,N-diethyl (meth)acrylamide, and diacetone (meth)acrylamide. These monofunctional (meth)acrylamides are contained in the N-substituted (meth)acrylamide (B) described above.

Examples of the vinyl group-containing monomer include N-vinylpyrrolidone, N-vinylcaprolactam, acrylonitrile, vinyl acetate, styrene, and vinyloxazoline. These monofunctional monomers may be used alone or in combination of two or more thereof.

The monofunctional oligomer, the polyfunctional oligomer, and the polymerizable polymer include, for example, linear and/or branched oligomers and polymers having a skeleton such as acrylic, ester, ether, urethane, and amide, and can be classified into urethane-based, epoxy-based, acrylic-based, and the like based on their main chain structure. When these are classified by weight average molecular weight (Mw), as those having an Mw of 1,000 or more and less than 10,000, oligomers containing the above polyfunctional (meth)acrylate and/or polyfunctional (meth)acrylamide can be exemplified, and as those having an Mw of 10,000 or more, the following polymerizable polymers can be exemplified: bifunctional polyurethane (meth)acrylate, polyfunctional polyurethane (meth)acrylate, bifunctional polyester (meth)acrylate, polyfunctional polyester (meth)acrylate, bifunctional polyether (meth)acrylate, polyfunctional polyether (meth)acrylate, bifunctional polyamide (meth)acrylate, polyfunctional polyamide (meth)acrylate, bifunctional poly(meth)acrylic acid ester (meth)acrylate, polyfunctional poly(meth)acrylic acid ester (meth)acrylate, bifunctional poly(meth)acrylic acid ester (meth)acrylamide, polyfunctional poly(meth)acrylic acid ester (meth)acrylamide, bifunctional poly (meth)acrylamide (meth)acrylate, polyfunctional poly (meth)acrylamide (meth)acrylate, bifunctional polystyrene (meth)acrylate, polyfunctional polystyrene (meth)acrylate, bifunctional polyacrylonitrile (meth)acrylate, polyfunctional polyacrylonitrile (meth)acrylate, bifunctional epoxy acrylate (bisphenol A type), polyfunctional epoxy acrylate (bisphenol A type), and the like. These oligomers or polymers may be used alone or in combination of two or more thereof.

In addition, from the viewpoint of easy availability as a commercial product, for the monofunctional or polyfunctional oligomers, urethane acrylates can be used, such as product names UV-3200B, UV-3000B, UV-6640B, UV-3700B, UV- 3310B, and UV-7000B manufactured by Mitsubishi Chemical Corporation, product names U-4HA and U-200PA manufactured by Shin-Nakamura Chemical Co., Ltd., product names EBECRYL245, EBECRYL1259, EBECRYL8210, EBECRYL284, and EBECRYL8402 manufactured by Daicel Cytec Co., Ltd., product names CN944, CN969, CN9002, and CN9029 manufactured by Sartomer, product names UN1255 and UN-5507 manufactured by Negami Chemical Industrial Co., Ltd., and product names AH-600 and UA-306I manufactured by Kyoeisha Chemical Co., Ltd., and UV curable urethane oligomers can be used, such as Quick Cure (registered trademark) 6100, Quick Cure (registered trademark) 7100, and Quick Cure (registered trademark) 8100 manufactured by KJ Chemicals Corporation.

The compound having an unsaturated bond may be a monomer or oligomer having an unsaturated bond, and is preferably the above-mentioned various monofunctional monomers, polyfunctional monomers, or oligomers. The compound having an unsaturated bond contained in the polymerizable composition of the present embodiment is preferably a monofunctional monomer having one unsaturated bond from the viewpoint of obtaining a thermoplastic product of polymerization used for adjusting physical properties such as viscosity, and is particularly preferably a monomer capable of introducing a crosslinking point from the viewpoint of increasing the cohesive force of the obtained product of polymerization. The content of these compounds having an unsaturated bond may be arbitrarily adjusted according to the specific use, but is preferably 1 to 99 wt% in total based on the total weight of the polymerizable composition according to the present embodiment.

The monomer capable of introducing the crosslinking point is a monomer having one or more reactive functional groups in the molecule, and examples thereof include functional group-containing (meth)acrylic monomers such as a hydroxyl group-containing (meth)acrylic monomer, a carboxyl group-containing (meth)acrylic monomer, an amino group-containing (meth)acrylic monomer, an acetoacetyl group-containing (meth)acrylic monomer, an isocyanate group-containing (meth)acrylic monomer, a glycidyl group-containing (meth)acrylic monomer, and an oxazoline group-containing vinyl monomer.

Examples of the hydroxyl group-containing (meth)acrylic monomer include: hydroxyalkyl (meth)acrylates such as 2-hydroxyethyl (meth)acrylate, 4-hydroxybutyl (meth)acrylate, 5-hydroxypentyl (meth)acrylate, 6-hydroxyhexyl (meth)acrylate, and 8-hydroxyoctyl (meth)acrylate; hydroxyalkyl (meth)acrylamides such as N-hydroxyethyl (meth)acrylamide and N-hydroxypropyl (meth)acrylamide; in addition to primary hydroxyl group-containing (meth)acrylic monomers such as 2-acryloyloxyethyl 2-hydroxyethyl phthalic acid and N-methylol (meth)acrylamide; secondary hydroxyl group-containing (meth)acrylic monomers such as 2-hydroxypropyl (meth)acrylate, 2-hydroxybutyl (meth)acrylate, 2-hydroxy-3-phenoxypropyl (meth)acrylate, and 3-chloro-2-hydroxypropyl (meth)acrylate; and a tertiary hydroxyl group-containing (meth)acrylic monomers such as 2,2-dimethyl-2-hydroxyethyl (meth)acrylate. Among these, hydroxyalkyl (meth)acrylates are preferably used.

Examples of the carboxyl group-containing (meth)acrylic monomer include monocarboxylic acids such as (meth)acrylic acid and crotonic acid, and dicarboxylic acids such as maleic acid, maleic anhydride, fumaric acid, citraconic acid, and itaconic acid. Among these, the (meth)acrylic acid is preferably used.

Examples of the amino group-containing (meth)acrylic monomer include: aminoalkyl (meth)acrylates such as N,N-dimethylaminoethyl (meth)acrylate, N,N-dimethylaminopropyl (meth)acrylate, N,N-di-t-butylaminoethyl (meth)acrylate, and N,N-diethylaminoethyl (meth)acrylate; and aminoalkyl (meth)acrylamides such as N,N-dimethylaminoethyl (meth)acrylamide, N,N-diethylaminoethyl (meth)acrylamide, and N,N-dimethylaminopropyl (meth)acrylamide.

Examples of the acetoacetyl group-containing (meth)acrylic monomer include 2-(acetoacetoxy)ethyl (meth)acrylate.

Examples of the glycidyl group-containing (meth)acrylic monomer include glycidyl (meth)acrylate, (meth)acrylamide glycidyl, N-hydroxyethyl (meth)acrylamide glycidyl ether, N-methyl-N-hydroxyethyl (meth)acrylamide glycidyl ether, N-ethyl-N-hydroxyethyl (meth)acrylamide glycidyl ether, N-propyl-N-hydroxyethyl (meth)acrylamide glycidyl ether, N-butyl-N-hydroxyethyl (meth)acrylamide glycidyl ether, N-hydroxypropyl (meth)acrylamide glycidyl ether, N-hydroxybutyl (meth)acrylamide glycidyl ether, N-hydroxypentyl (meth)acrylamide glycidyl ether, N-hydroxyhexyl (meth)acrylamide glycidyl ether, N-hydroxyheptyl (meth)acrylamide glycidyl ether, and N-hydroxyoctyl (meth)acrylamide glycidyl ether.

Examples of the oxazoline group-containing vinyl monomer include 2-vinyl-2-oxazoline, 4-methyl-2-vinyl-2-oxazoline, 5-methyl-2-vinyl-2-oxazoline, 4-ethyl-2-vinyl-2-oxazoline, 5-ethyl-2-vinyl-2-oxazoline, 4,4-dimethyl-2-vinyl-2-oxazoline, 4,4-diethyl-2-vinyl-2-oxazoline, 4,5-dimethyl-2-vinyl-2-oxazoline, 4,5-diethyl-2-vinyl-2-oxazoline, 2-isopropenyl-2-oxazoline, 4-methyl-2-isopropenyl-2-oxazoline, 5-methyl-2-isopropenyl-2-oxazoline, 4-ethyl-2-isopropenyl-2-oxazoline, 5-ethyl-2-isopropenyl-2-oxazoline, 4,4-dimethyl-2-isopropenyl-2-oxazoline, 4,4-diethyl-2-isopropenyl-2-oxazoline, 4,5-dimethyl-2-isopropenyl-2-oxazoline, and 4,5-diethyl-2-isopropenyl-2-oxazoline. In addition, 2-vinyl-2-oxazoline, 5-methyl-2-vinyl-2-oxazoline, and 4,4-dimethyl-2-vinyl-2-oxazoline having high reactivity are preferable, and 2-vinyl-2-oxazoline is most preferable. These monomers capable of introducing crosslinking points are not limited to one type, and a plurality of types may be used in combination.

In the present embodiment, the crosslinking agent is a compound capable of introducing a crosslinked structure into the cured product of the polymerizable composition, and examples thereof include: a monomer capable of introducing the above-described crosslinking point; an oligomer and a polymer capable of introducing a plurality of crosslinking points formed by polymerizing the monomer; a compound having two or more reactive functional groups in the molecule; and a polyfunctional monomer, a polyfunctional oligomer, and a polymerizable polymer having two or more unsaturated bonds in the above-described molecule. Note that in the present embodiment, when the monomer capable of introducing a crosslinking point is a monofunctional monomer having one or more reactive functionalities in the molecule, the monomer is handled as a monofunctional monomer. Examples of a crosslinking method using the crosslinking agent in the present embodiment include (1) a method of crosslinking the polymerizable composition or the product of polymerization of the polymerizable composition by further incorporation of a compound having a functional group (for example, an isocyanate group or a carboxyl group) that reacts with a reactive functional group (for example, a hydroxyl group or an amino group) contained in the polymerizable composition or the product of polymerization of the polymerizable composition, (2) a method of crosslinking the polymerizable composition or the product of polymerization of the polymerizable composition by incorporation of a polyfunctional monomer, a polyfunctional oligomer or a polymerizable polymer and by irradiation of active energy rays, and (3) a method of crosslinking the polymerizable composition or the product of polymerization of the polymerizable composition, by incorporation of the crosslinking agent such as the polyfunctional monomer, the polyfunctional oligomer, the polymerizable polymer, or the monomer capable of introducing a cross-linking point and by the irradiation of active energy rays and/or reaction of the crosslinking agent. Note that the crosslinking method (3) is an appropriate combination of the crosslinking methods (1) and (2).

In the crosslinking method (1), examples of the crosslinking agent (that is, the compound having the functional group that reacts with the reactive functional group contained in the polymerizable composition or the product of polymerization of the polymerizable composition) include an isocyanate compound, an epoxy compound, an aziridine compound, and a compound having a carboxyl group or an oxazoline group.

Examples of the isocyanate compound include aromatic isocyanates such as tolylene diisocyanate and xylene diisocyanate, alicyclic isocyanates such as isophorone diisocyanate, and aliphatic isocyanates such as hexamethylene diisocyanate. More specific examples of the isocyanate compound include: lower aliphatic polyisocyanates such as butylene diisocyanate and hexamethylene diisocyanate; alicyclic isocyanates such as cyclopentylene diisocyanate, cyclohexylene diisocyanate, and isophorone diisocyanate; aromatic diisocyanates such as 2,4-tolylene diisocyanate, 4,4'-diphenylmethane diisocyanate, and xylylene diisocyanate; and isocyanate adducts such as trimethylolpropane/tolylene diisocyanate trimer adduct (manufactured by Tosoh Corporation, product name: Coronate L), trimethylolpropane/hexamethylene diisocyanate trimer adduct (manufactured by Tosoh Corporation, product name: Coronate HL), and isocyanurate of hexamethylene diisocyanate (manufactured by Tosoh Corporation, product name: Coronate HX). These isocyanate compounds may be used alone or in combination of two or more thereof.

Examples of the epoxy compound include polyethylene glycol diglycidyl ether, polyglycerol polyglycidyl ether, glycerol diglycidyl ether, diglycidyl ether, trimethylolpropane triglycidyl ether, diglycidyl aniline, N,N,N',N'-tetraglycidyl-m-xylenediamine (manufactured by MITSUBISHI GAS CHEMICAL COMPANY, INC., product name: TETRAD-X), and 1,3-bis(N, N-diglycidylaminomethyl)cyclohexane (manufactured by MITSUBISHI GAS CHEMICAL COMPANY, INC., product name: TETRAD-C). These compounds may be used alone or in combination of two or more thereof.

Examples of the aziridine compound include product name: HDU, product name: TAZM, and product name: TAZO (all manufactured by Sogo Pharmaceutical Co., Ltd.) as commercially available products. These compounds may be used alone or in combination of two or more thereof.

Examples of the compound having a carboxyl group include: aromatic dicarboxylic acids such as phthalic acid, isophthalic acid, 1,4-naphthalenedicarboxylic acid, 2,6-naphthalenedicarboxylic acid, diphenyldicarboxylic acid, diphenoxyethanedicarboxylic acid, diphenyletherdicarboxylic acid, and diphenylsulfonedicarboxylic acid; alicyclic dicarboxylic acids such as 1,3-cyclopentanedicarboxylic acid, 1,3-cyclohexanedicarboxylic acid and 1,4-cyclohexanedicarboxylic acid; aliphatic dicarboxylic acids such as malonic acid, dimethylmalonic acid, succinic acid, 3,3-diethylsuccinic acid, glutaric acid, 2,2-dimethylglutaric acid, adipic acid, 2-methyladipic acid, trimethyl adipic acid, pimelic acid, azelaic acid, sebacic acid, and suberic acid; hydroxycarboxylic acids such as glycolic acid, 3-hydroxybutyric acid, 4-hydroxyvaleric acid, hydroxypropionic acid, hydroxy caproic acid, and hydroxybenzoic acid; and compounds having a dicarboxyl group derived from ester-forming derivatives thereof, anhydrides thereof, or the like. These compounds may be used alone or in combination of two or more thereof.

Examples of the compound having an oxazoline group include: alkylene bisoxazolines such as tetramethylene bisoxazoline and hexamethylene bisoxazoline; aromatic bisoxazolines such as 1,3-phenylene bis(2-oxazoline) and 1,4-bis(4,5-dihydro-2-oxazolyl)benzene; and compounds having an oxazoline group such as homopolymers of various oxazoline group-containing vinyl monomers described above and copolymers with compounds having an unsaturated bond. These compounds may be used alone or in combination of two or more thereof.

The content of the crosslinking agent (that is, a compound having two or more functional groups reactive with the reactive functional groups contained in the polymerizable composition or the product of polymerization in the molecule) in the crosslinking method (1) can be appropriately selected depending on balance with an amount and molecular weight of the reactive functional group contained in the polymerizable composition or the product of polymerization of the polymerizable composition, and further depending on the specific use, but is usually preferably 0.1 to 15 wt%, more preferably 0.5 to 10 wt%, and particularly preferably 1 to 5 wt% based on the total weight of the polymerizable composition. When the content is less than 0.1 wt%, crosslinking formation by the crosslinking agent is insufficient, and when the molded article after crosslinking reaction is a cured product of the adhesive composition and the glue composition, sufficient durability may not be obtained, and adhesive residue (contamination) tends to be caused in the adhesive composition. Further, when the molded article after the crosslinking reaction is a cured product of a coating agent, sufficient surface hardness may not be obtained, and when the molded article after the crosslinking reaction is a cured product of a three-dimensional modeling ink, that is, a three-dimensional shaped article, a shaped article having sufficient hardness and tensile strength may not be obtained. On the other hand, when the content is more than 15 wt%, if the molded article after the crosslinking reaction is the cured product of the adhesive composition and the glue composition, the adhesiveness to the substrate is also reduced along with reduction in flexibility, and sufficient adhesive strength and bond strength cannot be obtained. Further, when the molded article after the crosslinking reaction is the cured product of the three-dimensional modeling ink, that is, the three-dimensional shaped article, a shaped article having a sufficient elongation at break may not be obtained. Note that the crosslinking reaction in the crosslinking method (1) may be performed at normal temperature, but is preferably performed at a temperature of about 40°C to 120°C in order to promote the reaction.

In the crosslinking method (2), as the crosslinking agent (that is, the polyfunctional monomer, the polyfunctional oligomer, or the polymerizable polymer), those described above can be used. In addition, in the crosslinking method (2), the irradiation can be performed using various active energy rays described later. When ultraviolet rays (UV, UV-LED, and the like) or visible rays are used as the active energy rays, it is preferable to use a photopolymerization initiator in combination. When an electron beam (EB) or an energy beam having a wavelength shorter than that of the electron beam is used as the active energy ray, high energy is given, and thus the photopolymerization initiator may not be contained. In addition, when the unsaturated bond in the molecule of the polyfunctional monomer, the polyfunctional oligomer, or the polymerizable polymer is a self-initiating functional group such as a maleimide group, an allyl ether group, or a vinyl ether group, the photopolymerization initiator may not be contained.

The content of the crosslinking agent (that is, the polyfunctional monomer, the polyfunctional oligomer, or the polymerizable polymer) in the crosslinking method (2) is preferably 0.05 to 50 wt% based on the total weight of the polymerizable composition, and from the viewpoint of a good balance between the adhesiveness of the cured product

after crosslinking to various substrates and hardness and strength of the resulting cured product, the content is more preferably 0.1 to 30 wt%, and particularly preferably 0.5 to 20 wt%. When the content is less than 0.05 wt%, the crosslinking formation by the crosslinking agent is insufficient, and when the molded article after crosslinking reaction is the adhesive composition and the glue composition, sufficient durability may not be obtained, and the adhesive residue (contamination) tends to be caused in the adhesive composition. Further, when the molded article after the crosslinking reaction is the cured product of the coating agent, sufficient surface hardness may not be obtained, and when the molded article after the crosslinking reaction is the cured product of the three-dimensional modeling ink, that is, the three-dimensional shaped article, the shaped article having sufficient hardness and tensile strength may not be obtained. On the other hand, when the content is more than 50 wt%, if the molded article after the crosslinking reaction is the cured product of the adhesive composition and the glue composition, shrinkage due to curing specific to active energy ray curing tends to occur, which tends to cause peeling and cracking. In that case, of course, sufficient adhesive strength and bond strength cannot be obtained. Further, when the molded article after the crosslinking reaction is the cured product of the coating agent, peeling and cracking of a cured film which is the molded article tends to occur due to curing shrinkage. When the molded article after the crosslinking reaction is the three-dimensional shaped article, sufficient elongation at break may not be obtained.

The content of the crosslinking agent (that is, the polyfunctional monomer, the polyfunctional oligomer, or the polymerizable polymer, and the compound having two or more reactive functional groups in the molecule) in the crosslinking method (3) varies depending on a type of crosslinking agent used. The content of the polyfunctional monomer, the polyfunctional oligomer, or the polymerizable polymer is preferably 0.05 to 30 wt% based on the total weight of the polymerizable composition, and the content of the compound having two or more reactive functional groups in the molecule is preferably 0.1 to 10 wt% based on the total weight of the polymerizable composition. In addition, since the polyfunctional monomer, the polyfunctional oligomer, the polymerizable polymer, and the compound having two or more reactive functional groups in the molecule are mixed and used, the total content of these crosslinking agents is preferably 0.15 to 40 wt%, more preferably 0.2 to 35 wt%, and particularly preferably 0.5 to 30 wt% based on the total weight of the polymerizable composition. When the content is less than 0.15 wt%, the crosslinking formation by the crosslinking agent is insufficient, and when the molded article after crosslinking reaction is the cured product of the adhesive composition and the glue composition, sufficient durability may not be obtained, and the adhesive residue (contamination) tends to be caused in the adhesive composition. Further, when the molded article after the crosslinking reaction is a cured product of a coating agent, sufficient surface hardness may not be obtained, and when the molded article after the crosslinking reaction is a cured product of a three-dimensional modeling ink, that is, a three-dimensional shaped article, a shaped article having sufficient hardness and tensile strength may not be obtained. When the content is more than 40 wt%, if the molded article after the crosslinking reaction is the cured product of the adhesive composition and the glue composition, the adhesiveness to the substrate is also reduced along with reduction in flexibility, and sufficient adhesive strength and bond strength cannot be obtained. Further, when the molded article after the crosslinking reaction is the cured product of the three-dimensional modeling ink, that is, the three-dimensional shaped article, the shaped article having a sufficient elongation at break may not be obtained.

As described above, the polymerizable composition according to the present embodiment may further contain a non-polymerizable oligomer having a weight average molecular weight (Mw) of 1,000 or more and less than 10,000 and/or a non-polymerizable polymer having an Mw of 10,000 or more. Examples of the non-polymerizable oligomer and the non-polymerizable polymer include thermoplastic resins, rosin-based resins, and mixtures thereof. Examples of the thermoplastic resin include (meth)acrylic resins, cyclic polyolefin resins, cellulose resins, polyester resins, polyurethane resins, polysulfonic acid resins, ABS resins which are copolymers of acrylonitrile, butadiene, and styrene, polycarbonate resins, polyamide resins, and polyimide resins. Examples of the rosin-based resin include natural rosin such as gum rosin, and modified rosin resins such as hydrogenated rosin obtained by modifying the natural rosin, disproportionated rosin, rosin modified phenol resin, maleic acid modified rosin resin, maleated rosin, and esterified gum. These non-polymerizable oligomers and non-polymerizable polymers may be used alone or in combination of two or more thereof.

As described above, the polymerizable composition according to the present embodiment may further contain the polymerization initiator and/or the compound having an unsaturated bond. In this case, the polymerizable composition is further improved in curability and polymerizability by active energy rays and/or heat, and can be suitably used as an active energy ray-curable and/or thermosetting adhesive composition, glue composition, coating agent composition, ink composition, and the like. For example, as the adhesive composition, an adhesive layer can be formed by applying the polymerizable composition to a separator or various substrates described later and then curing the composition with active energy rays. In the present specification, polymerization of the polymerizable composition with active energy rays and heat is also referred to as hybrid polymerization. In the hybrid polymerization, the polymerization may be performed in the order of active energy rays and heat, or the polymerization may be performed in the order of heat and active energy rays.

The active energy ray is defined as an energy ray capable of decomposing a compound (photopolymerization initiator) that generates active species, to generate active species. Examples of such active energy rays include visible

light, ultraviolet rays, infrared rays, α rays, β rays, γ rays, X-rays, and electron beams (EB). When the electron beam is used as the active energy ray, the photopolymerization initiator may not be used. On the other hand, when the ultraviolet ray, the visible light, or the like is used, it is preferable to use the photopolymerization initiator. Irradiation with the active energy ray is preferably performed under an inert gas atmosphere such as nitrogen gas or carbon dioxide gas or under an atmosphere with a reduced oxygen concentration, but the polymerizable composition according to the present embodiment has N-substituted (meth)acrylamide (A), and thus has good curability, and can be sufficiently cured even under a normal air atmosphere. Irradiation temperature of the active energy ray is preferably 10°C to 200°C, and irradiation time is preferably 1 second to 60 minutes.

The photopolymerization initiator may be any substance (that is, photoradical polymerization initiator) that generates radicals by irradiating ultraviolet rays having an appropriate wavelength that can be a trigger for polymerization reaction thereof depending on a type of active energy ray reactive component. The photopolymerization initiator may be appropriately selected from ordinary photopolymerization initiators such as acetophenone-based, benzoin-based, benzophenone-based, and thioxanthone-based photopolymerization initiators, and as commercially available products, product names Omnirad 1116, Omnirad 1173, Omnirad 184, Omnirad 369, Omnirad 500, Omnirad 651, Omnirad 754, Omnirad 819, Omnirad 907, Omnirad 1300, Omnirad 1800, Omnirad 1870, Omnirad 2959, Omnirad 4265, Omnirad TPO, and the like manufactured by IGM Resins B.V, and product name Ubecryl P36 and the like manufactured by UCB can be used. These photopolymerization initiators may be used alone or in combination of two or more thereof.

The photoradical polymerization initiator is not particularly limited, and examples thereof include: benzoins such as benzoin, benzoin methyl ether, benzoin ethyl ether, benzoin isopropyl ether, benzoin isobutyl ether, and anisole methyl ether; acetophenones such as 4-(2-hydroxyethoxy)phenyl (2-hydroxy-2-propyl)ketone, α-hydroxy-α,α'-dimethylacetophenone, methoxyacetophenone, 2,2'-dimethoxy-2-phenylacetophenone, 2-hydroxy-2-cyclohexylacetophenone, 2,2-diethoxyacetophenone, 2,2-dimethoxy-2-phenylacetophenone, 1-hydroxycyclohexylphenyl ketone, 4-phenoxydichloroacetophenone, and 4-t-butyl-dichloroacetophenone; propiophenones such as 2-hydroxy-2-methylpropiophenone and 2-hydroxy-4'-isopropyl-2-methylpropiophenone; benzophenones such as benzophenone, methylbenzophenone, p-chlorobenzophenone, and p-dimethylaminobenzophenone; thioxanthones such as thioxanthone, 2-chlorthioxanthone, 2-ethylthioxanthone, 2-isopropylthioxanthone, 2,4-dichlorothioxanthone, 2,4-diethylthioxanthone, 2,4-diisopropylthioxanthone, and dodecylthioxanthone; acylphosphine oxides such as bis(2,4,6-trimethylbenzoyl)-phenylphosphine oxide, 2,4,6-trimethylbenzoyldiphenylphosphine oxide, bis(2,4,6-trimethylbenzoyl)-2,4-di-n-butoxyphenylphosphine oxide, and bis(2,6-dimethoxybenzoyl)-2,4,4-trimethylpentylphenylphosphine oxide; benzyl; dibenzosuberone; and α-acyloxime ester. Photoradical polymerization initiators may be used alone or in combination of two or more thereof.

The content of these photopolymerization initiators is usually 0.1 to 10 wt%, preferably 0.1 to 5 wt%, and more preferably 0.5 to 3 wt% based on the total weight of the polymerizable composition according to the present embodiment. When the content of the photopolymerization initiator is less than 0.1 wt%, sufficient curability cannot be obtained, and when the content is more than 10 wt%, performance such as strength of the cured product may be reduced.

Thermal polymerization of the polymerizable composition according to the present embodiment can be performed by a known method in the presence of a thermal polymerization initiator, and examples of the known method include methods such as an emulsion polymerization method, a solution polymerization method, a suspension polymerization method, and a bulk polymerization method. When the solution polymerization method is employed, a solvent that can be used is not particularly limited as long as it is a solvent that dissolves the product of polymerization obtained by polymerization, and examples of the solvent include: aromatic hydrocarbons such as benzene, toluene, ethylbenzene, and xylene; aliphatic hydrocarbons such as hexane, heptane, octane, decane, and cyclohexane; esters such as ethyl acetate, butyl acetate, and 2-hydroxyethyl acetate; aliphatic alcohols such as ethyl alcohol, n-propyl alcohol, and isopropyl alcohol; ketones such as acetone, methyl ethyl ketone, and methyl isobutyl ketone; acetonitrile; and N,N-dimethylformamide. These solvents may be used alone or in combination of two or more thereof. In particular, low-boiling ethyl acetate, methyl ethyl ketone, acetone, and the like are preferably used from the viewpoint of easy removal when the molded article is formed. The temperature and time of thermal polymerization vary depending on a thermal polymerization method to be employed and the thermal polymerization initiator to be used, but are usually calculated from a half-life of the initiator, and the temperature is usually preferably 60°C to 120°C, and the time is usually preferably 2 hours to 20 hours, and more preferably 5 hours to 10 hours.

Examples of the thermal polymerization initiator include thermal radical polymerization initiators, and azo compound catalysts such as azobisisobutyronitrile, azobisvaleronitrile, and dimethyl azobis(isobutyrate), peroxide-based catalysts such as benzoyl peroxide and hydrogen peroxide, persulfate catalysts such as ammonium persulfate and sodium persulfate, and the like can be used. The content of the thermal polymerization initiator is about 0.01 to 10 wt% based on the total weight of the polymerizable composition. Furthermore, a conventional radical polymerization technique such as adjustment of the molecular weight by a chain transfer agent can be applied.

As described above, the product of polymerization obtained by polymerizing the polymerizable composition according to the present embodiment by the above-described various methods may be further contained as a component of the polymerizable composition. The content of the product of polymerization of the polymerizable composition according

to the present embodiment is usually 1,000 to 2 million, preferably 5,000 to 1 million, and particularly preferably 10,000 to 500,000 in weight average molecular weight (Mw). When the Mw is in a range of 1,000 to 2 million, solution viscosity when the product of polymerization is dissolved in the solvent or a general-purpose low viscosity monomer is not too high or too low, so that the product of polymerization can be suitably handled, and processing accuracy of an adhesive sheet, a coating film, the three-dimensional shaped article, and the like is high. When the product of polymerization is diluted with ethyl acetate so that a solid content of the product of polymerization is 30%, the solution viscosity at 25°C is usually 10 to 100,000 mPa·s, preferably 500 to 10,000 mPa·s, and more preferably 1,000 to 5,000 mPa·s. The viscosity can be measured according to a cone plate viscometer method of JIS K5600-2-3.

A fifth embodiment of the present invention is the adhesive composition (hereinafter, also referred to as the adhesive). The adhesive composition according to the fifth embodiment contains the polymerizable composition or the product of polymerization of the polymerizable composition according to the first to fourth embodiments, or the polymerizable composition or the product of polymerization of the polymerizable composition, and the crosslinking agent. Since the polymerizable composition or the product of polymerization of the polymerizable composition according to the first to fourth embodiments contains the N-substituted (meth)acrylamide (A), the polymerizable composition or the product of polymerization of the polymerizable composition has sufficient cohesive force and adhesive strength as the adhesive composition, adhesiveness to various substrates, stain resistance, and also durability and yellowing resistance. Therefore, the polymerizable composition or the product of polymerization of the polymerizable composition can also be directly used as the adhesive composition. On the other hand, by crosslinking the adhesive composition containing the polymerizable composition or the product of polymerization of the polymerizable composition by the above-mentioned crosslinking methods (1) to (3) using the crosslinking agent, it is also possible to obtain the adhesive composition more excellent in stain resistance and durability.

The adhesive composition according to the fifth embodiment can be directly used as the adhesive layer after being applied to or molded on the separator or thesubstrate. On the other hand, it can also be used as the adhesive composition that forms the adhesive layer by being cured with active energy rays and/or heat, that is, the active energy ray-curable and/or thermosetting adhesive composition. Further, when an organic solvent is contained in the adhesive composition, the adhesive composition is coated or molded on the separator or thesubstrate, and then dried at a temperature of 60°C to 120°C for 1 minute to 30 minutes. As a method for applying the adhesive composition to the separator or the substrate, a conventionally known method can be used, and for example, a usual coating film forming method such as a spin coating method, a spray coating method, a knife coating method, a dipping method, a gravure roll, a reverse roll method, a screen printing method, or a bar coater method is used.

A multi-layer laminate product is obtained by laminating the adhesive layer including the adhesive composition according to the fifth embodiment and various substrates. Examples of a method for laminating the adhesive layer and various substrates include a transfer method and a roll-to-roll method. A thickness of the adhesive layer in the multi-layer laminate product is not particularly limited since it varies depending on various uses, but is usually 4 μm to 150 μm, and it is appropriate that the thickness is about 20 μm to 120 μm in the case of being used for an automobile member, and about 30 μm to 100 μm in the case of being used for an electronic material or an optical member.

Examples of the substrate include the substrates of various materials having a wide range of polarities from low to high polarity, such as the substrate of organic material, the substrate of inorganic material, and the substrate of the organic-inorganic hybrid material, and examples of the materials include polyolefins such as polyethylene and polypropylene, polyethylene terephthalate, polycarbonate, ABS resins which are acrylonitrile-butadiene-styrene copolymers, polyimide, polyamide, acrylic resins such as polymethyl methacrylate, metals such as steel, stainless steel, copper, and aluminum, glasses, and also hybrid materials in which silica fine particles which are inorganic materials are dispersed in polyimide which is an organic material. The use of the various base materials is not particularly limited, and examples of the use include those for electronic materials, optical members, and automobile members.

The adhesive layer constituting the multi-layer laminate product according to the fifth embodiment is molded from the polymerizable composition containing the N-substituted (meth)acrylamide (A) or the product of polymerization of the polymerizable composition, and the N-substituted (meth)acrylamide (A) has a hydrophobic substituent exhibiting wettability to the low polarity substrate and a hydrophilic (meth)acrylamide group exhibiting wettability to the high polarity substrate in the molecule, and can provide good adhesiveness from the low polarity substrate to the high polarity substrate. Further, high adhesive strength and stain resistance can be provided by exerting strong cohesive force derived from hydrogen bonds between amide groups between molecules of A. Furthermore, since the polymerizable composition or the product of polymerization of the polymerizable composition has high transparency and yellowing resistance, the adhesive layer obtained therefrom also has high transparency and yellowing resistance, and is suitably used in the optical field such as the adhesive for optical members and the adhesive sheet. The multi-layer laminate product including the adhesive layer having such characteristics and various substrates can be applied as an adhesive film or an adhesive sheet for electronic materials, optical members, and automobile members.

In the adhesive composition according to the fifth embodiment, the N-substituted (meth)acrylamide (A) contained in the adhesive composition can be brought in from the polymerizable composition containing A, and A can also be

brought in as a structural unit from the product of polymerization of the polymerizable composition, and a product of polymerization of the N-substituted (meth)acrylamide (A). The total content of the N-substituted (meth)acrylamide (A) and the structural unit of A in the adhesive is preferably 0.1 to 90 wt%, more preferably 1 to 70 wt%, and particularly preferably 5 to 60 wt% based on the total weight of the adhesive composition (excluding the solvent, the same applies hereinafter). Further, from the viewpoint of further improving wettability and adhesiveness to various substrates, the surface tension of the N-substituted (meth)acrylamide (A) is preferably 24.0 to 46.0 $mN\cdot m^{-1}$.

In the adhesive composition according to the fifth embodiment, from the viewpoint of improving the water resistance of the adhesive layer formed using the adhesive composition, the polymerization initiator, the compound having an unsaturated bond, and a non-polymerizable component (including non-polymerizable oligomer, non-polymerizable polymer, and the product of polymerization according to the second embodiment of the present invention), which are further contained, are preferably hydrophobic components. Further, in order to adjust a balance between hydrophilicity and hydrophobicity of the adhesive composition, the content of the monofunctional monomer (excluding the N-substituted (meth)acrylamide (A)) is preferably 10 to 90 wt%, more preferably 20 to 80 wt%, and particularly preferably 30 to 70 wt% based on the total weight of the adhesive composition. The total content of the crosslinking agent containing the polyfunctional monomer is preferably 1 to 30 wt%, more preferably 2 to 20 wt%, and particularly preferably 5 to 15 wt% based on the total weight of the adhesive composition. The total content of the non-polymerizable component is preferably 0.1 to 20 wt%, more preferably 0.5 to 15 wt%, and particularly preferably 1 to 10 wt% based on the total weight of the adhesive composition. Such an adhesive composition has high adhesiveness to various substrates, the adhesive layer formed from the adhesive composition has high adhesive strength, and it is possible to obtain the adhesive composition having excellent transparency, water resistance, stain resistance, yellowing resistance, and durability, and the multi-layer laminate product of the adhesive layer containing the adhesive composition and various substrate.

A sixth embodiment of the present invention is the glue composition (hereinafter, also referred to as an adhesive). The glue composition according to the sixth embodiment contains the polymerizable composition or the product of polymerization of the polymerizable composition according to the first to fourth embodiments, and the crosslinking agent. By containing the N-substituted (meth)acrylamide (A) and the crosslinking agent, the glue composition exhibits high bond strength tothe substrates of various materials having a wide range of polarities from low to high polarity, such as the substrate of organic material, the substrate of inorganic material, and the substrate of the organic-inorganic hybrid material, and impact resistance, and thus can be used as the glue composition for the same or different types of materials.

The same type of material refers to the same type of material among various materials such as resins, metals, glasses, and hybrid materials described above. In the same type of material, the surface tension of each material at 23°C is preferably 22.6 $mN\cdot m^{-1}$ to 59.0 $mN\cdot m^{-1}$. If the same type of material has a surface tension within the above range, high bond strength can be obtained. The surface tension of each material at 23°C can be measured according to JIS K 6768, and a minimum value of the surface tension that can be measured by this method is 22.6 $mN\cdot m^{-1}$. In the present specification, for convenience of calculating an absolute value of a difference in surface tension between different types of materials described later, the surface tension of the material in which a value of the surface tension measured by this method is less than 22.6 $mN\cdot m^{-1}$ is expressed as 22.6 $mN\cdot m^{-1}$.

The different types of materials refer to different types of materials among various materials such as resins, metals, glasses, and hybrid materials described above. In the different types of materials, the absolute value of the difference in surface tension at 23°C between two different types of materials bonded via the glue composition is preferably 37.0 $mN\cdot m^{-1}$ or less. Even when different materials are used as the different types of materials, their surface tensions may be the same, and thus the absolute value of the difference in surface tension is 0.0 $mN\cdot m^{-1}$ or more. If the different types of materials have the absolute value of the difference in surface tension within the above range, good adhesion can be obtained.

In the glue composition according to the sixth embodiment, the N-substituted (meth)acrylamide (A) contained in the glue composition can be brought in from the polymerizable composition containing A, and A can also be brought in as the structural unit from the product of polymerization of the polymerizable composition, and the product of polymerization of the N-substituted (meth)acrylamide (A). The total content of the N-substituted (meth)acrylamide (A) and the structural unit of A in the adhesive is preferably 1 to 95 wt%, more preferably 10 to 85 wt%, and particularly preferably 2 to 80 wt% based on the total weight of the adhesive (excluding the solvent). Further, from the viewpoint of further improving the bond strength between the same type of materials and between different types of materials, the surface tension of the N-substituted (meth)acrylamide (A) is preferably 24.0 to 46.0 $mN\cdot m^{-1}$.

In the glue composition according to the sixth embodiment, from the viewpoint of improving the water resistance of the cured product obtained by curing the glue composition, the polymerization initiator, the compound having an unsaturated bond, and the non-polymerizable component (including the non-polymerizable oligomer, the non-polymerizable polymer, and the product of polymerization according to the second embodiment of the present invention), which are further contained, are preferably hydrophobic components. Further, in order to adjust the balance between hydrophilicity and hydrophobicity and to improve the cohesive strength of the glue composition, the content of the monofunctional monomer (excluding the N-substituted (meth)acrylamide (A)) is preferably 1 to 85 wt%, more preferably 2 to 80 wt%, and particularly preferably 5 to 75 wt% based on the total weight of the glue composition. Furthermore, the content of the (meth)acrylate-based monomer as the monofunctional monomer is most preferably less than 50 wt% in order to sufficiently maintain the cohesive force derived from the (meth)acrylamide-based monomer.

In the glue composition according to the sixth embodiment, the total content of the crosslinking agent containing the polyfunctional monomer is preferably 1 to 50 wt%, more preferably 5 to 30 wt%, and particularly preferably 10 to 25 wt% based on the total weight of the glue composition. When the content is less than 1 wt%, the crosslinking formation by the crosslinking agent is insufficient, the cohesive force of the adhesive is insufficient, and sufficient impact resistance and heat resistance may not be obtained. On the other hand, when the content is more than 50 wt%, since crosslinking density of the adhesive after crosslinking is too high, the adhesiveness to the substrate tends to be reduced due to shrinkage, and as a result, the bond strength tends to be reduced. The crosslinking reaction using the crosslinking agent can be performed by the above-described crosslinking methods (1) to (3). Further, the total content of the non-polymerizable component is preferably 0.01 to 15 wt%, more preferably 0.1 to 10 wt%, and particularly preferably 0.5 to 8 wt% based on the total weight of the glue composition. Such a glue composition has high bond strength to various substrates, impact resistance, and water resistance, and is suitably used for bonding the same or different types of materials. In addition, the N-substituted (meth)acrylamide (A) used in the present invention has high yellowing resistance, and the glue composition according to the sixth embodiment can also be used as an adhesive of a retardation film or an optical film multi-layer laminate product such as a polarizing plate.

A seventh embodiment of the present invention is the cosmetic composition (hereinafter, also referred to as a cosmetic). The cosmetic composition according to the seventh embodiment contains the polymerizable composition or the product of polymerization of the polymerizable composition according to the first to fourth embodiments. The cosmetic composition can be used as a cosmetic composition having moisture resistance by containing the N-substituted (meth)acrylamide (A). The cosmetic composition can also be used as a cosmetic composition having emulsification stability due to well-balanced amphiphilicity of the N-substituted (meth)acrylamide (A). From the viewpoint of improving the moisture resistance and the emulsification stability, the surface tension of the N-substituted (meth)acrylamide (A) is preferably 24.0 to 46.0 $mN \cdot m^{-1}$.

The cosmetic composition according to the seventh embodiment may further contain other components depending on the use and dosage form, and the other components are not particularly limited. For example, when the cosmetic composition is used as a skin cosmetic, examples of the other components include various polymerization initiators, polyalkylene oxide macromonomers such as polyethylene oxide macromonomers, hydrocarbon oils for producing oil-in-water emulsified cosmetics, higher fatty acids, higher alcohols, synthetic ester oils, silicone oils, liquid oils and fats, solid oils and fats, waxes, oil phase components such as fragrances, water, water-soluble alcohols, and aqueous phase components such as thickeners. Further, when the cosmetic composition is used as a hair spray type hair cosmetic, examples of the other components include the polymerization initiator, a polymer surfactant, and a basic compound. In particular, by using tert-octylacrylamide, tert-butylacrylamide, or the like, the cosmetic composition is improved in solubility in liquefied petroleum gas (LPG), and thus can be suitably used (tert-octylacrylamide) or used in combination (tert-butylacrylamide) in aerosol products such as hair sprays using LPG as the hair cosmetic.

In the cosmetic composition according to the seventh embodiment, the N-substituted (meth)acrylamide (A) contained in the cosmetic composition can be brought in from the polymerizable composition containing A, and A can also be brought in as the structural unit from the product of polymerization of the polymerizable composition, and the product of polymerization of the N-substituted (meth)acrylamide (A). The total content of the N-substituted (meth)acrylamide (A) and the structural unit of A in the cosmetic composition is preferably 1 to 80 wt%, more preferably 5 to 70 wt%, and particularly preferably 10 to 60 wt% based on the total weight of the cosmetic composition. Such a cosmetic composition does not exhibit skin irritation, has moisture resistance or emulsification stability and stability over time, and is excellent in usability such as smoothness, stickiness resistance, texture, rich feeling, and quick familiarity.

An eighth embodiment of the present invention is the coating agent composition (hereinafter, also referred to as the coating agent). The coating agent composition according to the eighth embodiment contains the polymerizable composition or the product of polymerization of the polymerizable composition according to the first to fourth embodiments. By containing the N-substituted (meth)acrylamide (A), the coating agent composition has high wettability to the substrates of various materials having a wide range of polarities from low to high polarity, such as the substrate of organic material, the substrate of inorganic material, and the substrate of the organic-inorganic hybrid material. As a method for applying the coating agent composition to the substrate, a conventionally known method can be used. The coating agent composition is applied to the substrate and then cured with the active energy rays and/or heat described above. A thickness when the coating agent composition is applied to the substrate is not particularly limited, but is preferably a thickness sufficiently cured with the active energy rays and/or heat, and is generally 1 to 100 μm. An obtained cured product (a coating layer) has adhesiveness to the various substrates described above, and exhibits high pencil hardness and water resistance.

In the coating agent composition according to the eighth embodiment, the N-substituted (meth)acrylamide (A)

contained in the coating agent composition can be brought in from the polymerizable composition containing A, and A can also be brought in as the structural unit from the product of polymerization of the polymerizable composition, and the product of polymerization of the N-substituted (meth)acrylamide (A). The total content of the N-substituted (meth)acrylamide (A) and the structural unit of A in the coating agent is preferably 1 to 80 wt%, more preferably 5 to 70 wt%, and particularly preferably 10 to 60 wt% based on the total weight of the coating agent (excluding the solvent). Further, from the viewpoint of further improving adhesiveness to various substrates, the surface tension of the N-substituted (meth)acrylamide (A) is preferably 24.0 to 46.0 $mN \cdot m^{-1}$.

The coating agent composition according to the eighth embodiment preferably further contains the above-described crosslinking agent, and in this case, the pencil hardness and the water resistance of the coating layer are improved. From this point of view, the content of the crosslinking agent is preferably 5 to 70 wt%, more preferably 10 to 60 wt%, and particularly preferably 20 to 50 wt% based on the total weight of the coating agent composition. The coating agent composition may contain the photopolymerization initiator, the monofunctional monomer, the monofunctional oligomer, the non-polymerizable oligomer, and the non-polymerizable polymer described above as the other components. The content of the other components may be within a range not impairing the above-described characteristics of the coating agent composition, and is usually 0.1 to 20 parts by weight based on 100 parts by weight of the polymerizable composition. Such a coating agent composition has high wettability and adhesiveness to various substrates, and can be used to obtain the coating film exhibiting high surface hardness and water resistance by being cured.

A ninth embodiment of the present invention is the ink composition (hereinafter, also referred to as an ink). The ink composition according to the ninth embodiment contains the polymerizable composition or the product of polymerization of the polymerizable composition according to the first to fourth embodiments. The ink composition has high curability by containing the N-substituted (meth)acrylamide (A). As a method for applying the ink composition to the substrate, a conventionally known method can be used. An ink viscosity at 25°C is preferably less than 500 mPa·s, and from the viewpoint that the ink can be applied to the substrate by an inkjet method, more preferably less than 100 mPa·s. The ink composition is applied to the substrate and then cured with the active energy rays and/or heat described above to form an ink layer. In the obtained ink layer, from the viewpoint of further improving adhesiveness to various substrates, the surface tension of the N-substituted (meth)acrylamide (A) is preferably 24.0 to 46.0 $mN \cdot m^{-1}$.

In the ink composition according to the ninth embodiment, the N-substituted (meth)acrylamide (A) contained in the ink composition can be brought in from the polymerizable composition containing A, and A can also be brought in as the structural unit from the product of polymerization of the polymerizable composition, and the product of polymerization of the N-substituted (meth)acrylamide (A). The total content of the N-substituted (meth)acrylamide (A) and the structural unit of A in the ink is preferably 5 to 90 wt%, more preferably 10 to 85 wt%, and particularly preferably 15 to 80 wt% based on the total weight of the ink (excluding the solvent).

The ink composition according to the ninth embodiment may further contain the above-described crosslinking agent, and in this case, the curability, surface dryness, and water resistance of the ink layer are improved. From this point of view, the content of the crosslinking agent is preferably 1 to 50 wt%, more preferably 5 to 45 wt%, and particularly preferably 10 to 40 wt% based on the total weight of the ink composition. The ink composition may further contain the photopolymerization initiator, the monofunctional monomer, the monofunctional oligomer, the non-polymerizable oligomer, and the non-polymerizable polymer described above as the other components. The content of the other components may be within a range not impairing the above-described characteristics of the ink composition, and is usually 0.1 to 30 parts by weight based on 100 parts by weight of the polymerizable composition. Such an ink composition has high adhesiveness to various substrates, and is excellent in printing characteristics such as pigment dispersibility, surface dryness, ejection stability, and sharpness, and by using the ink composition, an ink having high curability, yellowing resistance, and water resistance can be obtained.

A tenth embodiment of the present invention is a three-dimensional modeling ink composition. The three-dimensional modeling ink composition according to the tenth embodiment contains the polymerizable composition or the product of polymerization of the polymerizable composition according to the first to fourth embodiments, or the polymerizable composition or the product of polymerization of the polymerizable composition, and the crosslinking agent. Since the polymerizable composition or the product of polymerization of the polymerizable composition according to the first to fourth embodiments contains the N-substituted (meth)acrylamide (A), the three-dimensional modeling ink composition has high curability and curing shrinkage resistance, and a cured product thereof has high strength and water resistance and is excellent in modeling accuracy. The three-dimensional modeling ink composition is formed into a predetermined shape pattern, and at the same time or immediately after being formed, the three-dimensional modeling ink composition is cured by irradiation with active energy rays and/or by heat to form a thin film, and the thin film is laminated to shape the three-dimensional shaped article. A shaping method is not particularly limited, and examples thereof include a stereolithography in which the three-dimensional modeling ink composition is discharged by an inkjet method and cured by irradiation with the active energy ray. In this case, from the viewpoint of stable discharge, the viscosity of the three-dimensional modeling ink composition at 25°C is preferably 1 to 200 mPa·s, and a discharge temperature is preferably in a range of 20 to 100°C. From the viewpoint that the obtained three-dimensional shaped

article can exhibit good appearance such as glossiness and denseness, the surface tension of the N-substituted (meth)acrylamide (A) is preferably 24.0 to 46.0 $mN\cdot m^{-1}$.

In the three-dimensional modeling ink composition according to the tenth embodiment, the N-substituted (meth)acrylamide (A) contained in the three-dimensional modeling ink composition can be brought in from the polymerizable composition containing A, and A can also be brought in as the structural unit from the product of polymerization of the polymerizable composition, and the product of polymerization of the N-substituted (meth)acrylamide (A). The total content of the N-substituted (meth)acrylamide (A) and the structural unit of A in the three-dimensional modeling ink composition is preferably 1 to 80 wt%, more preferably 2 to 70 wt%, and particularly preferably 5 to 60 wt% based on the total weight of the three-dimensional modeling ink.

The three-dimensional modeling ink composition according to the tenth embodiment preferably further contains the crosslinking agent described above, and in this case, the three-dimensional shaped article more excellent in strength, water resistance, and heat resistance can be formed. From this point of view, the content of the crosslinking agent is preferably 1 to 50 wt%, more preferably 5 to 40 wt%, and particularly preferably 10 to 30 wt% based on the total weight of the three-dimensional modeling ink composition. Further, the three-dimensional modeling ink composition may further contain the photopolymerization initiator, the monofunctional monomer, the monofunctional oligomer, the non-polymerizable oligomer, and the non-polymerizable polymer described above as the other components. The content of the other components may be within a range not impairing the above-described characteristics of the three-dimensional modeling ink composition, and is usually 0.1 to 20 parts by weight based on 100 parts by weight of the polymerizable composition. From such a three-dimensional modeling ink composition, the three-dimensional shaped article having high strength, heat resistance, and water resistance can be accurately formed.

Various additives other than those described above can be blended in the polymerizable compositions according to the first to fourth embodiments as necessary. Examples of the additives include thermopolymerization inhibitors, antioxidants, ultraviolet sensitizers, preservatives, phosphoric acid ester-based and other flame retardants, surfactants, antistatic agents, colorants such as pigments and dyes, fragrances, antifoaming agents, fillers, silane coupling agents, surface tension modifiers, plasticizers, surface lubricants, leveling agents, softeners, organic fillers, inorganic fillers, and silica particles. These additives may be used alone or in combination of two or more thereof. The content of these additives is not particularly limited as long as it does not adversely affect the properties exhibited by various molded articles of the polymerizable composition or of the product of polymerization of the polymerizable composition, but is preferably 5 wt% or less based on the total weight of the polymerizable composition.

Various additives other than those described above can be blended in the polymerizable compositions according to the fifth to tenth embodiments as necessary. Examples of the additives include thermopolymerization inhibitors, antioxidants, ultraviolet sensitizers, preservatives, phosphoric acid ester-based and other flame retardants, surfactants, antistatic agents, colorants such as pigments and dyes, fragrances, antifoaming agents, fillers, silane coupling agents, surface tension modifiers, plasticizers, surface lubricants, leveling agents, softeners, organic fillers, inorganic fillers, and silica particles. These additives may be used alone or in combination of two or more thereof. The content of these additives is not particularly limited as long as it does not adversely affect the properties exhibited by various molded articles obtained from the various compositions, but is preferably 30 wt% or less based on the total weight of the composition. In addition, if necessary, water, the organic solvent, and a mixture thereof can be used as a solvent or a diluent. The content of such a solvent is not particularly limited as long as it does not adversely affect the properties exhibited by various molded articles obtained from the various compositions, but is preferably 95 wt% or less based on the total weight of the composition.

[Examples]

Hereinafter, the present invention will be described in more detail with reference to Examples and Comparative Examples, but the present invention is not limited to the following Examples. Abbreviations of constituent components described in Examples and Comparative Examples are as follows. Further, in the following description, "parts" and "%" are all based on weight unless otherwise specified.

(1) N-substituted (meth)acrylamide (A)

CHAA: N-cyclohexylacrylamide (registered trademark "Kohshylmer") (solid at normal temperature, surface tension: 32.3 $mN\cdot m^{-1}$)
CHMAA: N-cyclohexyl-N-methylacrylamide (registered trademark "Kohshylmer") (liquid at normal temperature, surface tension: 32.5 $mN\cdot m^{-1}$)
ACP: N-acryloylpiperidine (registered trademark "Kohshylmer") (liquid at normal temperature, surface tension: 35.7

mN·m$^{-1}$)
ACMP: N-acryloyl-4-methylpiperidine (registered trademark "Kohshylmer") (liquid at normal temperature, surface tension: 31.6 mN·m$^{-1}$)
ACDMP: N-acryloyl-3,5-dimethylpiperidine (registered trademark "Kohshylmer") (liquid at normal temperature, surface tension: 28.6 mN·m$^{-1}$)
NOAA: n-octylacrylamide (registered trademark "Kohshylmer") (wax state at normal temperature, surface tension value: 30.0 mN·m$^{-1}$)
TOAA: tert-octylacrylamide (registered trademark "Kohshylmer") (solid at normal temperature, surface tension: 29.1 mN·m$^{-1}$)
EHAA: N-(2-ethylhexyl)acrylamide (registered trademark "Kohshylmer") (liquid at normal temperature, surface tension: 29.7 mN·m$^{-1}$)
DEHAA: N,N-di-2-ethylhexylacrylamide (registered trademark "Kohshylmer") (liquid at normal temperature, surface tension: 31.0 mN·m$^{-1}$)
LMAA: N-lauryl methacrylamide (registered trademark "Kohshylmer") (solid at normal temperature, surface tension: 31.2 mN·m$^{-1}$)
OLAA: N-oleylacrylamide (registered trademark "Kohshylmer") (liquid at normal temperature, surface tension: 32.3 mN·m$^{-1}$)
STAA: N-stearylacrylamide (registered trademark "Kohshylmer") (solid at normal temperature, surface tension: 31.9 mN·m$^{-1}$)
AAPB: 3-acrylamidophenylboronic acid (registered trademark "Kohshylmer") (solid at normal temperature, surface tension: 24.0 mN·m$^{-1}$)
DPAA: dopamine acrylamide (registered trademark "Kohshylmer") (solid at normal temperature, surface tension: 36.5 mN·m$^{-1}$)
PHAM: phenyl acrylamide (registered trademark "Kohshylmer") (solid at normal temperature, surface tension: 45.3 mN·m$^{-1}$)

(2) Monofunctional monomer

TBAA: tert-butylacrylamide
BA: butyl acrylate
2EHA: 2-ethylhexyl acrylate
STA: stearyl acrylate
OLA: oleyl acrylate
EA: ethyl acrylate
HEA: hydroxyethyl acrylate
4HBA: 4-hydroxybutyl acrylate
PEA: phenoxyethyl acrylate
IBOA: isobornyl acrylate
THFA: tetrahydrofurfuryl acrylate
AAc: acrylic acid
VOZO: 2-vinyl -2-oxazoline (registered trademark "Kohshylmer")
MHAGE: N-methyl-N-hydroxyethylacrylamide glycidyl ether (registered trademark "Kohshylmer")
GA: glycidyl acrylate (registered trademark "Kohshylmer")
"HEAA": hydroxyethyl acrylamide (registered trademarks "Kohshylmer", "HEAA")
"DMAA": dimethylacrylamide (registered trademarks "Kohshylmer", "DMAA")
"DEAA": diethylacrylamide (registered trademarks "Kohshylmer", "DEAA")
"NIPAM": isopropylacrylamide (registered trademarks "Kohshylmer", "NIPAM")
DAAM: diacetone acrylamide (registered trademark "Kohshylmer")

(3) Polyfunctional monomer and the like (Polyfunctional monomer and oligomer)

PETA: pentaerythritol triacrylate
DPHA: dipentaerythritol hexaacrylate
HDDA: 1,6-hexanediol diacrylate

TPGDA: tripropylene glycol diacrylate
UV-3000B: bifunctional urethane acrylate (SHIKOH manufactured by Mitsubishi Chemical Corporation)
UV-6640B: bifunctional urethane acrylate (SHIKOH manufactured by Mitsubishi Chemical Corporation)
"Quick Cure" 7100: UV-curable urethane oligomer (registered trademark "Quick Cure" manufactured by KJ Chemicals Corporation)
"Quick Cure" 8100: UV-curable urethane oligomer (registered trademark "Quick Cure" manufactured by KJ Chemicals Corporation)

(4) Others

O-184: Omnirad 184 (photopolymerization initiator, manufactured by IGM Resins B.V)
O-1173: Omnirad 1173 (photopolymerization initiator, manufactured by IGM Resins B.V)
TPO: Omnirad TPO (photopolymerization initiator, manufactured by IGM Resins B.V)
HDI: hexamethylene diisocyanate (crosslinking agent)
HHPA: hexahydrophthalic anhydride (crosslinking agent)
AIBN: azobisisobutyronitrile (radical polymerization initiator)
V-601: azobis(isobutyric acid) dimethyl (radical polymerization initiator)
KE-359: hydrogenated rosin (tackifier, non-polymerizable polymer, manufactured by Arakawa Chemical Industries, Ltd.)
VS-1063: styrene acrylic resin (non-polymerizable oligomer, manufactured by SEIKO PMC CORPORATION)
VS-1057: acrylic resin (non-polymerizable polymer, manufactured by SEIKO PMC CORPORATION)
50HB-55: polyoxyethylene(2)polyoxypropylene(2)butyl ether (m=2, n=2, molecular weight 240) (manufactured by Sanyo Chemical Industries, Ltd.)
50HB-100: polyoxyethylene(5)polyoxypropylene(5)butyl ether (m=5, n=5, molecular weight 540) (manufactured by Sanyo Chemical Industries, Ltd.)
50HB-260: polyoxyethylene(10)polyoxypropylene(7)butyl ether (m=10, n=7, molecular weight 880) (manufactured by Sanyo Chemical Industries, Ltd.)
AMP: 2-amino-2-methyl-1-propanol
PME4000: BLEMMER PME-4000 (manufactured by NOF CORPORATION)

(5) Substrate

PE: polyethylene plate and film (surface tension: 22.6 $mN \cdot m^{-1}$)
PP: polypropylene plate and film (surface tension: 22.6 $mN \cdot m^{-1}$)
PC: polycarbonate plate and film (surface tension: 34.0 $mN \cdot m^{-1}$)
ABS: acrylonitrile-butadiene-styrene copolymer synthetic resin plate (surface tension: 34.0 $mN \cdot m^{-1}$)
PI: polyimide plate and film (surface tension: 40.0 $mN \cdot m^{-1}$)
PMMA: polymethyl methacrylate plate and film (surface tension: 36.0 $mN \cdot m^{-1}$)
PET: easily adhesive polyethylene terephthalate plate and film (surface tension: 59.0 $mN \cdot m^{-1}$)
SPCC: cold-rolled steel sheet (surface tension: 45.0 $mN \cdot m^{-1}$)
SST: stainless steel plate (surface tension: 40.0 $mN \cdot m^{-1}$)
Cu: copper plate (surface tension: 38.0 $mN \cdot m^{-1}$)
Al: aluminum plate (surface tension: 36.0 $mN \cdot m^{-1}$)
GL: transparent glass plate (surface tension: 40.0 $mN \cdot m^{-1}$)
PI-silica: silica fine particle-dispersed polyimide plate and film (surface tension: 40.0 $mN \cdot m^{-1}$)

Example 1 (Production and evaluation of product of polymerization of N-substituted (meth)acrylamide (A))

In a 500 mL flask equipped with a thermometer, a stirrer, a reflux condenser, and a nitrogen inlet tube, 30 g of CHAAas N-substituted (meth)acrylamide (A), 44 g of 2EHA, 20 g of "NIPAM", 5 g of HEA, 1 g of AIBN, and 100 g of ethyl acetate as a solvent were added, and the mixture was uniformly mixed at room temperature, then purged with nitrogen for 30 minutes, and the reaction solution was heated to 70°C, and subjected to a polymerization reaction for 8 hours. After completion of the reaction, ethyl acetate was added to the reaction solution to prepare a polymer solution having a solid content of 30%. The viscosity of the polymer solution at 25°C was measured according to JIS K5600-2-3

using a cone plate viscometer (RE550 manufactured by Toki Sangyo Co., Ltd.) and was 4250 mPa s. In addition, 30 g of the polymer solution was taken out, and the volatile component in the solution was completely removed to obtain a polymer. Thereafter, the obtained polymer was dissolved in tetrahydrofuran (THF) to prepare a THF solution of a polymer having a concentration of 0.5 wt%, and the solution was allowed to stand overnight. Thereafter, the THF solution of the polymer was filtered through a 0.45 μm membrane filter, gel permeation chromatography (GPC) measurement was performed using the filtrate (Prominence GPC system manufactured by Shimadzu Corporation, Shodex KF-806L column, eluent THF), and the weight average molecular weight (Mw) of the polymer was calculated to be 960,000 from a polystyrene equivalent value.

(Evaluation of water resistance of product of polymerization)

Similarly, 30 g of the polymer solution was taken out, the volatile component in the solution was completely removed, and dried under vacuum at 60°C for 24 hours to obtain a dried polymer. Thereafter, 5 g of the dried polymer was accurately weighed using a plastic petri dish (previously weighed) to determine a weight in dry state of the product of polymerization. The petri dish containing the polymer was placed in a thermo-hygrostat, and after a lapse of 24 hours and 48 hours under conditions of 30°C and a humidity of 90%, the weight immediately after being taken out from the thermo-hygrostat was weighed, and it was confirmed that the polymer had reached a saturated water absorption state to determine a weight in the saturated water absorption state of the product of polymerization. A saturated water absorption was calculated according to the following formula, and the water resistance of the product of polymerization was evaluated in four grades as follows, and results are shown in Table 1.

$$\text{saturated water absorption (\%)} = (\text{weight in saturated water absorption state - weight in dry state})/\text{weight in dry state} \times 100\%$$

Excellent (E): saturated water absorption is 5% or more
Good (G): saturated water absorption is more than 5% but 7% or less
Fair (F): saturated water absorption is more than 7% but 10% or less
Poor (P): saturated water absorption is more than 10%

Examples 2 to 12 and Comparative Examples 1 to 3 (Production of product of polymerization of N-substituted (meth)acrylamide (A), and other polymers)

The types and contents of N-substituted (meth)acrylamide, monofunctional monomer, and polymerization initiator were changed as shown in Table 1, and polymerization reactions of Examples 2 to 12 and Comparative Examples 1 to 3 were performed in the same manner as in Example 1 to obtain polymer solutions having a solid content of 30%. The viscosity of the obtained polymer solution and the molecular weight of the polymer were measured in the same manner as in Example 1, and the results are shown in Table 1.

Table 1

| | | A | | Monofunctional monomer | | Polymerization initiator | | Molecular weight | Solution viscosity | Water resistance of product of polymerization |
|---|---|---|---|---|---|---|---|---|---|---|
| | | Type | wt% | Type | wt% | Type | wt% | (Mw, 10,000) | (mPa·s) | |
| Examples | 1 | CHAA | 30 | 2EHA | 44 | AIBN | 1 | 96 | 4250 | G |
| | | | | "NIPAM" | 20 | | | | | |
| | | | | HEA | 5 | | | | | |
| | 2 | OLAA | 60 | BA | 28 | AIBN | 2 | 80 | 3150 | E |
| | | | | IBOA | 10 | | | | | |
| | 3 | EHAA | 10 | 2EHA | 45 | AIBN | 5 | 112 | 4650 | G |
| | | | | DAAM | 40 | | | | | |
| | 4 | LMAA | 40 | BA | 15 | AIBN | 1 | 88 | 3800 | E |
| | | DEHAA | 40 | AAc | 4 | | | | | |
| | 5 | TOAA | 30 | 2EHA | 49 | AIBN | 1 | 80 | 3000 | E |
| | | | | "DEAA" | 20 | | | | | |
| | 6 | CHMAA | 20 | THFA | 38 | AIBN | 2 | 95 | 4000 | E |
| | | | | TBAA | 30 | | | | | |
| | | | | 4HBA | 10 | | | | | |
| | 7 | OLAA | 85 | VOZO | 0.5 | AIBN | 1.5 | 33 | 1400 | E |
| | | DPAA | 5 | BA | 8 | | | | | |
| | 8 | ACMP | 40 | 2EHA | 49.5 | V-601 | 0.5 | 76 | 2700 | E |
| | | NOAA | 5 | "HEAA" | 5 | | | | | |
| | 9 | ACDMP | 1 | "DMAA" | 50 | AIBN | 0.1 | 120 | 4500 | F |
| | | | | 2EHA | 47 | | | | | |
| | | | | MHAGE | 1.9 | | | | | |
| | 10 | OLAA | 0.8 | BA | 45 | AIBN | 9.2 | 72 | 2600 | F |
| | | | | "DEAA" | 40 | | | | | |
| | | | | GA | 5 | | | | | |
| | 11 | ACP | 50 | 2EHA | 35 | V-601 | 1 | 66 | 1780 | E |
| | | AAPB | 5 | EA | 9 | | | | | |
| | 12 | STAA | 20 | BA | 45 | V-601 | 2 | 55 | 1700 | E |
| | | PHAA | 5 | TBAA | 23 | | | | | |
| | | | | "HEAA" | 5 | | | | | |
| Comparative Examples | 1 | | | TBAA | 50 | AIBN | 2 | 115 | 3600 | F |
| | | | | BA | 40 | | | | | |
| | | | | 4HBA | 8 | | | | | |
| | 2 | | | BA | 35 | AIBN | 5 | 84 | 3300 | P |
| | | | | "DMAA" | 60 | | | | | |
| | 3 | | | BA | 30 | AIBN | 1 | 90 | 3500 | F |
| | | | | OLA | 60 | | | | | |
| | | | | "NIPAM" | 9 | | | | | |

Examples 13 to 28 and Comparative Examples 4 to 7 (Preparation and evaluation of polymerizable composition)

The N-substituted (meth)acrylamide (A) used in the present embodiment and other components were weighed in the proportion shown in Table 2, and uniformly mixed at room temperature to prepare polymerizable compositions of Examples and Comparative Examples. The transparency and wettability to various substrates of the obtained polymerizable composition were evaluated by the following methods, and the results are shown in Table 2. Further, the polymerizable compositions of Examples 15, 16, 18, and 21 are polymerizable resin compositions for thermal polymerization, and are respectively Examples 4, 2, 8, and 3 shown in Table 1, and characteristics of products of polymerization are shown in Table 1. The polymerizable resin composition other than that for thermal polymerization is a polymerizable resin composition for active energy ray curing, curability and water resistance of the obtained cured product were evaluated by the following methods, and the results are shown in Table 2. Note that Example 14 is a method of curing by irradiating electron beams (EB) instead of ultraviolet rays. As an EB irradiation device, Curetron EBC-200-AA3 manufactured by Nisshin High Voltage Corporation was used (acceleration voltage: 200 kV, irradiation dose: 20 kGy).

Table 2

| | | Polymerizable composition | | | | | | | | | | Transparency | Wettability | | | | | Curability | Cured product water resistance |
|---|---|---|---|---|---|---|---|---|---|---|---|---|---|---|---|---|---|---|---|
| | | A | | Monofunctional monomer | | Crosslinking agent | | Polymerization initiator | | Others | | | PP | PMMA | GL | Al | PI-silica | | |
| | | Type | wt% | Type | wt% | Type | wt% | Type | wt% | Type | wt% | | | | | | | | |
| Examples | 13 | EHAA | 70 | THFA | 27 | PETA | 2 | TPO | 1 | | | E | G | E | E | E | G | G | E |
| | 14 | CHMAA<br>OLAA | 20<br>80 | | | | | | | | | E | E | E | E | E | E | G | E |
| | 15 | LMAA<br>DEHAA | 40<br>40 | BA<br>AAc | 15<br>4 | | | AIBN | 1 | | | G | G | E | G | G | E | - | - |
| | 16 | OLAA | 60 | BA<br>IBOA | 28<br>10 | | | AIBN | 2 | | | E | E | E | E | E | E | - | - |
| | 17 | ACMP | 50 | BA | 40 | Quick Cure 8100 | 8 | TPO | 2 | | | G | E | E | E | E | G | E | E |
| | 18 | ACMP<br>NOAA | 40<br>5 | 2EHA<br>"HEAA" | 49.5<br>5 | | | V-601 | 0.5 | | | E | G | E | E | E | E | - | - |
| | 19 | TOAA<br>ACP | 10<br>10 | "DMAA"<br>BA | 25<br>35 | PETA | 15 | O-184 | 3 | VS-1063 | 2 | G | G | E | E | E | E | E | E |
| | 20 | STAA<br>ACDMP | 15<br>20 | IOBA<br>THFA<br>"HEAA" | 13<br>35<br>5 | UV-3000B | 20 | O-184 | 2 | | | G | G | E | E | E | E | E | E |
| | 21 | EHAA | 10 | 2EHA<br>DAAM | 45<br>40 | | | AIBN | 5 | | | G | G | E | E | G | E | - | - |
| | 22 | DEHAA | 10 | "DMAA"<br>AAc<br>PEA | 20<br>5<br>40 | Quick Cure 7100 | 10 | O-1173 | 5 | Example 6 product of polymerization | 10 | G | G | E | G | G | E | E | E |
| | 23 | NOAA<br>OLAA | 5<br>30 | "DEAA"<br>2EHA<br>4HBA | 15<br>30<br>10 | | | O-1173 | 10 | | | E | G | E | E | E | G | G | E |
| | 24 | ACP | 1 | BA<br>"DEAA"<br>HEA | 45<br>25<br>4 | TPGDA | 20 | O-184 | 5 | | | E | G | G | G | G | G | G | G |
| | 25 | CHAA | 0.5 | 2EHA<br>"DEAA"<br>4HBA | 40<br>35<br>8 | UV-3000B | 10 | O-184 | 1.5 | KE-359 | 5 | E | F | G | G | G | F | G | G |
| | 26 | ACDMP<br>AAPB | 60<br>3 | BA<br>"NIPAM" | 25<br>5 | UV-3000B | 5 | O-184 | 2 | | | E | E | E | E | G | G | G | E |
| | 27 | LMAA<br>PHAM | 15<br>15 | THFA<br>OLA<br>AAc | 35<br>10<br>5 | Quick Cure 7100 | 12 | TPO | 3 | Example 8 product of polymerization | 5 | G | G | E | G | G | G | E | E |
| | 28 | DPAA<br>ACMP | 5<br>40 | PEA<br>THFA | 7<br>30 | DPHA | 0.5 | TPO | 2.5 | Example 2 product of polymerization | 15 | G | G | E | G | E | G | E | E |
| Comparative Examples | 4 | | | TBAA<br>BA | 45<br>40 | PETA | 10 | TPO | 3 | VS-1057 | 2 | F | P | F | P | P | P | P | F |
| | 5 | | | "DEAA"<br>2EHA<br>4HBA | 33<br>40<br>5 | UV-3000B | 20 | O-184 | 2 | | | G | P | F | F | P | P | F | P |
| | 6 | | | DAAM<br>THFA | 40<br>40 | DPHA | 10 | O-1173 | 5 | VS-1063 | 5 | F | P | G | F | F | P | F | P |
| | 7 | | | HEA<br>"DMAA"<br>PEA | 5<br>15<br>40 | TPGDA | 30 | O-1173 | 5 | VS-1057 | 5 | G | P | F | F | P | P | F | P |

(Transparency evaluation)

The obtained various polymerizable compositions were allowed to stand at 23°C overnight, the state of the composition was visually observed, and the transparency was evaluated in four grades as follows.

Excellent (E): high transparency and no cloudiness or separation is observed
Good (G): high transparency but slight cloudiness is observed
Fair (F): no layer separation but cloudy
Poor (P): cloudy and further separated into layers

(Wettability evaluation)

The obtained various polymerizable compositions were applied to various substrates with a bar coater (RDS

3), the degree of cissing of the coating film was visually observed, and the wettability was evaluated in four grades as follows.

Excellent (E): no cissing and coating film is uniform
Good (G): very little cissing but coating film is almost uniform
Fair (F): some cissing but coating film is almost uniform as a whole
Poor (P): many cissing and coating film is non-uniform

(Curability evaluation)

The curability was measured by placing a 100 μm thick PET film (polyester film COSMOSHINE A4100 manufactured by Toyobo Co., Ltd.) in close contact on a horizontally placed glass plate so that an easily adhesive surface of the PET film was a surface, applying the polymerizable resin composition for active energy ray curing prepared in each of Examples and Comparative Examples using a bar coater No. 30, and then further overlaying thereon a light release PET film (polyester film E7002 manufactured by Toyobo Co., Ltd.) having a thickness of 50 μm, and curing the resin composition by irradiating ultraviolet rays with a predetermined integrated light quantity (apparatus: manufactured by ITEC SYSTEM Co., Ltd., desktop batch-type UV-LED curing apparatus MUVBA-0.3 × 0.3 × 0.5, wavelength: 405 nm, illuminance (UV-V): 50 $mW/cm^2$). Thereafter, the release PET film was removed to obtain test pieces of the cured products for evaluating curability for Examples and Comparative Examples. Tack on the surface of the obtained cured product was evaluated, and the following evaluation was performed with the integrated light quantity at which the tack disappeared.

Excellent (E): tack disappears when integrated light quantity is less than 200 $mJ/cm^2$
Good (G): tack disappears when integrated light quantity is 200 $mJ/cm^2$ or more and less than 500 $mJ/cm^2$
Fair (F): tack disappears when integrated light quantity is 500 $mJ/cm^2$ or more and less than 1000 $mJ/cm^2$
Poor (P): tack disappears when integrated light quantity is 1000 $mJ/cm^2$ or more (including a case where tack does not disappear)

(Evaluation of water resistance of cured product)

A silicone spacer (length 30 mm × width 15 mm × thickness 1 mm) was set on a glass plate (length 50 mm × width 50 mm × thickness 5 mm), and the polymerizable resin composition for active energy ray curing prepared in each of Examples and Comparative Examples was poured into the spacer and cured by ultraviolet irradiation (700 $mW/cm^2$, 2000 $mJ/cm^2$) to prepare a cured sheet. The obtained sheet was cut into 3 cm square, dried under vacuum at 60°C for 24 hours, and accurately weighed as a dry sheet to determine a weight in dry state of the cured product. The dry sheet was immersed in deionized water at 30°C, and after a lapse of 24 hours and 48 hours, the weight immediately after being taken out from the deionized water was weighed, and it was confirmed that the sheet had reached a saturated water absorption state to determine a weight in the saturated water absorption state of the cured product. The saturated water absorption was calculated according to the following formula, and the water resistance of the cured product was evaluated in four grades as follows.

$$\text{saturated water absorption (\%)} = (\text{weight in saturated water absorption state} - \text{weight in dry state})/\text{weight in dry state} \times 100\%$$

Excellent (E): saturated water absorption is 5% or more
Good (G): saturated water absorption is more than 5% but 7% or less
Fair (F): saturated water absorption is more than 7% but 10% or less
Poor (P): saturated water absorption is more than 10%

Examples 29 to 70 and Comparative Examples 8 to 20 (Preparation and evaluation of adhesive composition)

(Examples 29 to 40 and Comparative Examples 8 to 10)

Using solutions of the product of polymerization obtained in Examples 1 to 12 and Comparative Examples 1 to 3, a heavy release separator (silicone-coated PET film) was coated so that the thickness after drying was 25 μm, and dried at 90°C for 2 minutes to form an adhesive layer. Subsequently, the film was placed in an environment of a temperature

of 23°C and a relative humidity of 50% for 1 day to obtain a test adhesive sheet (type a-1). Further, using a prepared test adhesive sheet (having an unsaturated bond derived from an oleyl group in the corresponding product of polymerization) of type a in Examples 30, 35, and 38 and Comparative Example 10, the sheet was irradiated with ultraviolet rays to obtain a test adhesive sheet (type a-2) (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 700 mW/$cm^2$, integrated light quantity: 1000 mJ/$cm^2$).

(Examples 41 to 48 and Comparative Example 11)

The solutions of the product of polymerization obtained in Examples 1, 4, 6, 8, and 12 and Comparative Example 1, and HDI as the crosslinking agent were weighed to have the solid contents shown in Table 4, and mixed uniformly. In the same manner as described above, the mixture was applied to a PET film so that the thickness after drying was 25 μm, and dried at 90°C for 2 minutes to form the adhesive layer. Thereafter, the film was aged in a thermostatic bath at 40°C for 3 days, and placed in an environment at a temperature of 23°C and a relative humidity of 50% for 1 day to obtain a test adhesive sheet (type b-1). Further, the solutions of the product of polymerization obtained in Examples 7, 9, and 10, and polyacrylic acid (PAAc manufactured by FUJIFII,M Wako Pure Chemical Corporation, average molecular weight 5,000) as the crosslinking agent were weighed to have the solid contents shown in Table 4, and mixed uniformly. In the same manner as described above, the mixture was applied to a PET film so that the thickness after drying was 25 μm, and dried at 90°C for 2 minutes to form the adhesive layer. Thereafter, the film was aged in a thermostatic bath at 40°C for 3 days, and placed in an environment at a temperature of 23°C and a relative humidity of 50% for 1 day to obtain a test adhesive sheet (type b-2).

(Examples 49 to 53 and Comparative Examples 12 and 13)

The solutions of the product of polymerization obtained in Examples 2, 4, 5, 7, 8, and 11 and Comparative Examples 2 and 3 were weighed, and a monofunctional monomer, a polyfunctional monomer and/or a polyfunctional oligomer as the crosslinking agent, a photopolymerization initiator, and other components were weighed in predetermined amounts shown in Table 5, and mixed uniformly. In the same manner as described above, the mixture was applied to a PET film so that the thickness after drying was 25 μm, and dried at 90°C for 2 minutes to form the adhesive layer. Thereafter, the film was irradiated with ultraviolet rays to be cured (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 700 mW/$cm^2$, integrated light quantity: 1000 mJ/$cm^2$), and placed in an environment at a temperature of 23°C and a relative humidity of 50% for 1 day to obtain a test adhesive sheet (type c).

(Examples 54 to 65 and Comparative Examples 14 to 17)

Using the polymerizable composition obtained in Examples 13, 14, 17, 19, 20, and 22 to 28 and Comparative Examples 4 to 7, a heavy release separator (silicone-coated PET film) was coated, and the film was bonded to a light release separator (silicone-coated PET film) so that the adhesive layer has a thickness of 25 μm using a desktop type roll laminator (RSL-382S manufactured by Royal Sovereign) so as not to entrain air bubbles, and was irradiated with ultraviolet rays (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 700 mW/$cm^2$, integrated light quantity: 1000 mJ/$cm^2$) to prepare an optical transparent adhesive sheet (type d).

(Examples 66 to 72 and Comparative Examples 18 to 20)

The solutions of the product of polymerization or the polymerizable compositions obtained in Examples 1, 6, 8, 12 to 14, 19, and 24 and Comparative Examples 1, 4, and 5, HDI or a polyfunctional monomer and/or a polyfunctional oligomer as the crosslinking agent, a photopolymerization initiator, and other components were weighed in predetermined amounts shown in Table 7, and mixed uniformly. An adhesive sheet was prepared in the same manner as in the type c (in the case of using the product of polymerization) or the type d (in the case of using the polymerizable composition), and cured with ultraviolet rays. Thereafter, the film was aged in a thermostatic bath at 40°C for 3 days, and placed in an environment at a temperature of 23°C and a relative humidity of 50% for 1 day to obtain a test adhesive sheet (type e).

Characteristics of various adhesive sheets prepared were evaluated by the following methods, and the results are shown in Tables 3 to 7.

(Transparency evaluation of adhesive sheet)

A total light transmittance of a glass substrate was measured according to JIS K 7105 using a haze meter (NDH-2000 manufactured by Nippon Denshoku Industries Co., Ltd.). The adhesive layer was transferred to the glass substrate under conditions of a temperature of 23°C and a relative humidity of 50%, and a total light transmittance of the glass substrate and the adhesive layer was measured. Thereafter, a transmittance of the glass plate was subtracted to calculate a transmittance of the adhesive layer itself, and the transparency was evaluated in four stages as follows.

Excellent (E): transmittance is 90% or more
Good (G): transmittance is 85% or more and less than 90%
Fair (F): transmittance is 50% or more and less than 85%
Poor (P): transmittance is less than 50%

(Adhesiveness evaluation)

The active energy ray-curable adhesive composition prepared above was applied to various plate-like base materials (substrates), and the substrate was bonded to a light release separator (silicone-coated PET film) so that the adhesive layer has a thickness of 5 μm using a desktop type roll laminator (RSL-382S manufactured by Royal Sovereign) so as not to entrain air bubbles, and was irradiated with ultraviolet rays (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 700 $mW/cm^2$, integrated light quantity: 2000 $mJ/cm^2$). Thereafter, the light release separator was peeled off to obtain an adhesive sheet including the adhesive layer and the substrate. Using the obtained adhesive sheet, 100 squares of 1 mm square were prepared according to JIS K 5600, and the number of squares in which the adhesive layer remained on the substrate side when the cellophane tape was attached and peeled off at once was counted, and the adhesiveness was evaluated according to the following criteria.

Excellent (E): 100 pieces were not peeled off
Good (G): 95 to 99 pieces were not peeled off
Fair (F): 70 to 94 pieces were not peeled off
Poor (P): 0 to 69 pieces were not peeled off

(Adhesive strength evaluation)

Under the conditions of a temperature of 23°C and a relative humidity of 50%, the adhesive layer was transferred to various film-like or plate-like substrates, pressure-bonded by reciprocating a pressure roller having a weight of 2 kg twice, and left for 30 minutes in the same atmosphere. Thereafter, 180° peel strength (N/25 mm) was measured at a peeling rate of 300 mm/min according to JIS Z 0237 using a tensile tester (apparatus name: Tensilon RTA-100 manufactured by ORIENTEC Co., Ltd.).

Excellent (E): 30 (N/25 mm) or more
Good (G): 15 (N/25 mm) or more and less than 30 (N/25 mm)
Fair (F): 8 (N/25 mm) or more and less than 15 (N/25 mm)
Poor (P): less than 8 (N/25 mm)

(Stain resistance (Reworkability) evaluation)

An adhesive sheet was prepared in the same manner as in measurement of the adhesive strength described above, left at 80°C for 24 hours, and then contamination of a surface of a substrate film (a residue state of the adhesive layer (adhesive)) after the adhesive sheet was peeled off was visually observed.

Excellent (E): no contamination (no adhesive residue)
Good (G): very slight contamination
Fair (F): slight contamination
Poor (P): contamination (adhesive residue)

(Yellowing resistance evaluation)

An adhesive sheet was prepared in the same manner as in the measurement of the adhesive strength described

above, set in a xenon fade meter (SC-700-WA manufactured by Suga Test Instruments Co., Ltd.), and irradiated with ultraviolet rays having an intensity of 70 mW/cm$^2$ for 120 hours, and then discoloration of the adhesive layer on the adhesive sheet was visually observed.

Excellent (E): yellowing cannot be visually observed at all
Good (G): yellowing can be visually observed very slightly
Fair (F): yellowing can be visually observed
Poor (P): obvious yellowing can be visually observed

(Durability evaluation)

An adhesive sheet was prepared in the same manner as in the measurement of the adhesive strength described above, and held for 100 hours under conditions of a temperature of 85°C and a relative humidity of 85%, and then the presence or absence of occurrence of lifting or peeling of the adhesive layer, air bubbles, and cloudiness was visually observed and evaluated.

Excellent (E): transparent, and no lifting, peeling, or air bubbles are generated
Good (G): slight cloudiness, but no lifting, peeling, or air bubbles are generated
Fair (F): slight cloudiness, or lifting, peeling, and air bubbles
Poor (P): extreme cloudiness, or lifting, peeling, and air bubbles

Table 3

| | | Adhesive composition | | Type of adhesive sheet | Transparency | Adhesiveness | | | | | | | Adhesive strength | | | | | Stain resistance | | | Yellowing resistance | Durability |
|---|---|---|---|---|---|---|---|---|---|---|---|---|---|---|---|---|---|---|---|---|---|---|
| | | Type of product of polymerization | wt% | | | PE | PP | PMMA | GL | Al | Cu | PI-silica | PET | PC | PI | PE | SST | PP | PET | Al | | |
| Examples | 29 | Example 1 | 100 | a-1 | G | G | E | E | E | E | G | G | E | G | G | G | E | G | G | G | E | G |
| | 30 | Example 2 | 100 | a-1 | G | E | E | E | E | E | G | G | E | E | E | E | E | E | E | E | G | E |
| | | | | a-2 | E | E | E | E | E | E | E | G | E | G | G | G | E | G | E | G | G | E |
| | 31 | Example 3 | 100 | a-1 | E | G | G | E | E | E | E | E | E | G | G | G | E | E | E | G | G | G |
| | 32 | Example 4 | 100 | a-1 | E | G | E | G | G | G | G | G | E | E | G | G | G | G | E | E | E | G |
| | 33 | Example 5 | 100 | a-1 | E | G | E | E | E | E | E | E | E | E | E | E | E | E | E | E | E | E |
| | 34 | Example 6 | 100 | a-1 | E | G | G | E | E | E | E | E | E | E | E | G | E | E | E | G | E | E |
| | 35 | Example 7 | 100 | a-1 | E | E | E | E | G | E | E | E | E | G | G | G | E | G | G | E | G | G |
| | | | | a-2 | E | E | E | E | G | E | E | E | E | E | E | E | E | G | G | E | G | E |
| | 36 | Example 8 | 100 | a-1 | G | G | E | G | E | E | E | G | G | G | G | G | G | G | E | G | E | E |
| | 37 | Example 9 | 100 | a-1 | E | G | E | E | E | E | E | E | E | E | E | E | E | G | G | E | E | E |
| | 38 | Example 10 | 100 | a-1 | G | G | G | E | E | G | G | E | E | E | E | E | E | E | E | E | E | G |
| | | | | a-2 | G | G | G | E | E | G | G | E | G | E | G | G | E | E | G | E | E | E |
| | 39 | Example 11 | 100 | a-1 | E | E | E | E | E | E | E | E | E | E | E | E | E | G | E | G | E | E |
| | 40 | Example 12 | 100 | a-1 | E | E | E | E | E | E | E | E | E | E | E | E | E | G | E | G | E | E |
| Comparative Examples | 8 | Comparative Example 1 | 100 | a-1 | G | P | P | F | F | P | F | P | P | P | P | P | F | F | P | P | F | P |
| | 9 | Comparative Example 2 | 100 | a-1 | G | F | F | F | F | P | F | P | P | P | P | F | F | P | P | P | F | F |
| | 10 | Comparative Example 3 | 100 | a-1 | F | F | P | F | F | P | F | F | F | F | P | F | F | P | F | F | F | F |
| | | | | a-2 | F | F | P | F | F | P | F | F | P | F | P | P | F | F | P | F | P | P |

Table 4

| | | Adhesive composition | | | | Type of adhesive sheet | Transparency | Adhesiveness | | | | Adhesive strength | | | Stain resistance | | | Yellowing resistance | Durability |
|---|---|---|---|---|---|---|---|---|---|---|---|---|---|---|---|---|---|---|---|
| | | Type of product of polymerization | wt% | Crosslinking agent type | wt% | | | PP | PMMA | GL | Al | PET | PC | SST | PP | PET | Al | | |
| Examples | 41 | Example 1 | 97 | HDI | 3 | b-1 | G | E | E | E | E | E | G | E | G | G | G | E | G |
| | 42 | Example 4 | 98 | HDI | 2 | b-1 | E | G | E | E | E | E | G | E | E | E | G | G | G |
| | 43 | Example 6 | 80 | HDI | 20 | b-1 | G | G | E | G | G | G | G | G | E | E | E | G | E |
| | 44 | Example 8 | 95 | HDI | 5 | b-1 | E | E | E | E | E | E | E | E | E | E | E | E | E |
| | 45 | Example 12 | 90 | HDI | 10 | b-1 | E | G | E | E | E | E | E | E | E | G | G | E | E |
| | 46 | Example 7 | 99.9 | PAAc | 0.1 | b-2 | E | E | G | E | E | G | G | G | G | E | G | G | E |
| | 47 | Example 9 | 99 | PAAc | 1 | b-2 | E | E | E | E | E | E | E | E | G | E | E | E | E |
| | 48 | Example 10 | 92 | PAAc | 8 | b-2 | E | E | E | E | E | E | E | E | G | G | G | E | E |
| Comparative Examples | 11 | Comparative Example 11 | 95 | HDI | 5 | b-1 | F | P | F | F | P | P | P | F | P | P | P | F | P |

Table 5

| | | Adhesive composition | | | | | | Transparency | Adhesiveness | | | | | Adhesive strength | | | | Stain resistance | | | Yellowing resistance | Durability |
|---|---|---|---|---|---|---|---|---|---|---|---|---|---|---|---|---|---|---|---|---|---|---|
| | | Type of product of polymerization | wt% | Crosslinking agent type | wt% | Other types | wt% | | PP | PMMA | GL | Cu | PI-silica | PET | PC | SST | PI | PP | PET | Al | | |
| Examples | 49 | Example 2 | 45 | PETA | 13 | "DMAA" | 40 | E | E | E | E | G | E | E | E | E | G | E | E | E | E | E |
| | | | | | | TPO | 2 | | | | | | | | | | | | | | | |
| | 50 | Example 4 | 10 | "Quick Cure" 7100 | 40 | "DEAA" | 35 | E | G | E | E | E | G | E | E | G | E | E | E | E | E | G |
| | | | | DPHA | 10 | O-1173 | 5 | | | | | | | | | | | | | | | |
| | 51 | Example 7 | 5 | HDDA | 30 | THFA | 40 | E | G | E | E | G | G | E | E | E | G | E | G | E | E | G |
| | | | | UV-3000B | 10 | O-184 | 10 | | | | | | | | | | | | | | | |
| | | | | TPGDA | 5 | | | | | | | | | | | | | | | | | |
| | 52 | Example 8 | 50 | "Quick Cure" 8100 | 5 | DAAM | 20 | E | E | E | G | E | E | E | E | G | E | E | E | G | E | E |
| | | Example 5 | 10 | PETA | 5 | O-1173 | 5 | | | | | | | | | | | | | | | |
| | 53 | Example 11 | 90 | DPHA | 9 | TPO | 1 | G | E | E | G | E | E | E | E | G | E | E | G | G | G | E |
| Comparative Examples | 12 | Comparative Example 2 | 50 | PETA | 10 | THFA | 25 | P | P | P | P | F | P | F | P | P | P | P | F | P | F | P |
| | | | | UV-3000B | 10 | O-1173 | 5 | | | | | | | | | | | | | | | |
| | 13 | Comparative Example 3 | 10 | HDDA | 30 | PEA | 38 | F | P | F | P | P | P | P | F | P | P | P | P | F | P | F |
| | | | | UV-3000B | 20 | TPO | 2 | | | | | | | | | | | | | | | |

Type of adhesive sheet: c

Table 6

| | | Adhesive composition | | Transparency | Adhesiveness | | | | | Adhesive strength | | | | Stain resistance | | | Yellowing resistance | Durability |
|---|---|---|---|---|---|---|---|---|---|---|---|---|---|---|---|---|---|---|
| | | Polymerizable composition type | wt% | | PP | PMMA | GL | Al | PI-silica | PET | PC | SST | PI | PP | PET | Al | | |
| Examples | 54 | Example 13 | 100 | E | G | E | E | E | G | E | E | E | G | E | E | E | E | E |
| | 55 | Example 14 | 100 | E | E | E | E | E | E | E | E | G | E | E | E | E | G | G |
| | 56 | Example 17 | 100 | G | E | E | E | G | G | E | E | E | G | E | G | E | E | G |
| | 57 | Example 19 | 100 | G | G | E | G | E | E | E | E | G | E | E | E | G | E | E |
| | 58 | Example 20 | 100 | G | G | E | E | E | E | E | E | E | G | E | E | E | E | E |
| | 59 | Example 22 | 100 | G | E | E | G | G | G | E | E | G | E | E | E | E | E | G |
| | 60 | Example 23 | 100 | E | G | E | G | E | G | E | E | G | E | E | G | G | G | E |
| | 61 | Example 24 | 100 | E | G | G | G | E | E | E | E | G | E | E | E | G | E | E |
| | 62 | Example 25 | 100 | E | F | G | G | G | F | E | G | G | F | G | G | G | E | G |
| | 63 | Example 26 | 100 | E | E | E | G | G | G | E | E | G | E | E | E | G | E | E |
| | 64 | Example 27 | 100 | G | G | E | G | G | G | E | E | G | E | E | E | E | E | G |
| | 65 | Example 28 | 100 | G | G | E | G | E | G | E | E | G | E | E | G | G | G | E |
| Comparative Examples | 14 | Comparative Example 4 | 100 | P | P | P | P | F | P | F | P | P | P | P | F | P | F | P |
| | 15 | Comparative Example 5 | 100 | P | P | F | P | F | P | P | P | P | F | P | F | P | F | P |
| | 16 | Comparative Example 6 | 100 | F | P | P | P | F | P | F | P | P | P | P | F | P | F | P |
| | 17 | Comparative Example 7 | 100 | F | P | F | P | P | P | P | F | P | P | P | P | F | P | F |

Type of adhesive sheet: d

Table 7

| | | Adhesive composition | | | | | | Transparency | | | | | | Adhesive strength | | | | Stain resistance | | | Yellowing resistance | Durability |
|---|---|---|---|---|---|---|---|---|---|---|---|---|---|---|---|---|---|---|---|---|---|---|
| | | Type of product of polymerization or polymerizable composition | wt% | Crosslinking agent type | wt% | Other types | wt% | | PP | PMMA | GL | Cu | PI-silica | PET | PC | SST | PI | PP | PET | Al | | |
| Examples | 66 | Example 1 | 30 | PETA<br>HDI | 20<br>10 | "DEAA"<br>O-184 | 35<br>5 | E | E | E | E | G | E | E | E | E | G | E | E | E | E | E |
| | 67 | Example 6 | 20 | "Quick Cure" 7100<br>DPHA | 30<br>20 | PEA<br>O-1173 | 20<br>10 | E | G | E | E | E | G | E | E | E | G | E | E | E | E | E |
| | 68 | Example 8<br>Example 19 | 0.5<br>10 | UV-3000B<br>HDI<br>HDDA | 47<br>0.5<br>20 | "DMAA"<br>THFA<br>O-1173 | 40<br>25<br>2 | E | G | E | E | E | G | E | E | G | G | E | E | G | E | G |
| | 69 | Example 12 | 50 | "Quick Cure" 8100<br>HDI | 25<br>5 | "DMAA"<br>O-184 | 15<br>5 | E | E | E | E | G | E | E | E | G | E | E | E | E | E | E |
| | 70 | Example 13 | 5 | UV-3000B<br>TPGDA | 25<br>22 | KE-359<br>"DEAA"<br>O-184 | 10<br>30<br>3 | E | G | E | E | E | G | E | E | E | G | E | E | E | E | G |
| | 71 | Example 14 | 40 | DPHA | 35 | "NIPAM"<br>O-1173 | 20<br>5 | E | E | E | E | G | E | E | E | G | E | E | E | G | E | E |
| | 72 | Example 24 | 80 | PETA | 5 | VS-1057<br>O-1173 | 12<br>3 | E | G | E | E | E | E | E | E | E | E | E | E | E | E | E |
| Comparative Examples | 18 | Comparative Example 1 | 50 | UV-3000B<br>HDI | 20<br>5 | "DMAA"<br>O-184 | 20<br>5 | P | P | P | P | P | P | F | P | P | P | P | F | P | F | P |
| | 19 | Comparative Example 4 | 50 | UV-3000B | 5 | "DMAA"<br>O-1173 | 40<br>5 | F | P | P | P | P | P | F | P | P | P | P | F | P | P | P |
| | 20 | Comparative Example 5 | 40 | HDDA | 30 | BA<br>TPO | 38<br>2 | F | P | F | P | P | P | P | F | P | P | P | P | F | P | F |

Type of adhesive sheet: e

Examples 73 to 82 and Comparative Examples 21 to 24 (Preparation and evaluation of adhesive composition)

The products of polymerization obtained in Examples 1 to 4, 6, 8, 9, and 11 and Comparative Examples 1 and 2 and/or the polymerizable compositions obtained in Examples 13, 14, 19, 20, and 25 to 27 and Comparative Examples 4 and 5, the crosslinking agent, and other components were weighed to have the solid content shown in Table 8, and were uniformly mixed at room temperature to obtain a uniform mixture. The mixture was uniformly applied to any one of two plate-like substrates of the same or different types having a length of 100 mm, a width of 25 mm, a thickness of 1 mm. Note that when a solvent was contained in the mixture, the mixture was applied in a larger amount so that the thickness after drying was about the same as that in the case of no solvent, and dried at 90°C for 2 minutes. Thereafter, according to JIS K 6850, the other one of the plate-like substrates was placed on the applied mixture, and bonded so that an overlapping region was 12.5 mm long × 25 mm wide, and the thickness of the adhesive layer was adjusted to 100 μm by using a spacer to prepare a bonded test piece. Thereafter, UV or EB irradiation was performed from an upper surface of the bonded transparent or translucent substrate in the same manner as in the preparation of the adhesive layer. Note that in Table 8, in Examples in which UV or EB is described as a curing method, test pieces after irradiation with UV rays or EB rays were used as adhesive test pieces. In Examples in which UV heat or EB heat is described as the curing method, test pieces after irradiation with UV rays or EB rays were further heated at 40°C for 72 hours, and the obtained test pieces were used as the adhesive test pieces. In Example in which heat is described as the curing method, a test piece prepared without irradiation with UV rays or EB rays was heated at 40°C for 72 hours, and the obtained test piece was used as the adhesive test piece. Further, using the obtained adhesive test piece, the bond strength and impact resistance were evaluated by the following method, and the results are shown in Table 8.

(Bond strength evaluation)

Using the obtained adhesive test piece, the tensile shear strength was measured according to JIS K 6850 under the condition of a tensile speed of 10 mm/min using Tensilon RTA-100 (manufactured by ORIENTEC Co., Ltd.) as a tester.

Excellent (E): tensile shear strength is 20MPa or more
Good (G): tensile shear strength is 15MPa or more and less than 20MPa
Fair (F): tensile shear strength is 10MPa or more and less than 15MPa
Poor (P): tensile shear strength is less than 10MPa

(Impact resistance evaluation)

Using the obtained adhesive test piece, the impact peeling adhesive strength was measured according to JIS K 6855 using an impact tester No. 511 (manufactured by MYS-TESTER Co., Ltd.).

Excellent (E): impact peeling adhesive strength is 20 $KJ/m^2$ or more
Good (G): impact peeling adhesive strength is 15 $KJ/m^2$ or more and less than 20 $KJ/m^2$
Fair (F): impact peeling adhesive strength is 10 $KJ/m^2$ or more and less than 15 $KJ/m^2$
Poor (P): impact peeling adhesive strength is less than 10 $KJ/m^2$

Table 8

| | No. | Glue composition: Polymerizable composition or product of polymerization – Type | wt% | Crosslinking agent – Type | wt% | Others – Type | wt% | Curing method | Substrate/substrate (difference in surface tension) | Bond strength | Impact resistance |
|---|---|---|---|---|---|---|---|---|---|---|---|
| Examples | 73 | Example 2 | 50 | DPHA | 10 | IBOA | 15 | UV | PET/PET ($0.0mN \cdot m^{-1}$) | E | E |
| | | | | UV-6640B | 20 | TPO | 5 | | | | |
| | 74 | Example 1 | 40 | TPGDA | 35 | STA | 12 | UV heat | PE/PMMA ($13.4mN \cdot m^{-1}$) | E | G |
| | | | | HDI | 10 | TPO | 3 | | PMMA/Al ($0.0mN \cdot m^{-1}$) | G | E |
| | 75 | Example 8 | 20 | PETA | 45 | DAAM | 25 | UV | PC/PI ($6.0 mN \cdot m^{-1}$) | G | E |
| | | | | HDI | 2 | O-1173 | 8 | | | | |
| | 76 | Example 11 | 30 | UV-6640B | 20 | DEAA | 20 | EB heat | PET/Al ($23.0 mN \cdot m^{-1}$) | E | G |
| | | Example 9 | 30 | | | | | | PP/ABS ($11.4mN \cdot m^{-1}$) | G | E |
| | 77 | Example 13 | 15 | TPGDA | 40 | TBAA | 10 | EB | PI/Cu ($2.0mN \cdot m^{-1}$) | G | E |
| | | | | "Quick Cure" 8100 | 20 | "DMAA" | 15 | | | | |
| | 78 | Example 14 | 35 | UV-6640B | 30 | | | UV heat | PP/GL ($17.4mN \cdot m^{-1}$) | E | E |
| | | Example 19 | 35 | | | | | | PET/PP ($36.4mN \cdot m^{-1}$) | E | G |
| | 79 | Example 20 | 5 | TPGDA | 30 | VS-1063 | 5 | UV | PMMA/SST ($4.0mN \cdot m^{-1}$) | G | G |
| | | Example 3 | 5 | "Quick Cure" 8100 | 50 | O-184 | 5 | | | | |
| | 80 | Example 25 | 80 | HDI | 5 | "HEAA" | 5 | UV heat | PC/SPCC ($11.0mN \cdot m^{-1}$) | E | G |
| | | Example 4 | 5 | | | | | | | | |
| | 81 | Example 26 | 2 | PETA | 30 | VS-1057 | 10 | UV | PP/GL ($17.4mN \cdot m^{-1}$) | E | G |
| | | | | UV-6640B | 15 | TBAA | 35 | | | | |
| | | | | | | O-184 | 8 | | | | |
| | 82 | Example 27 | 10 | DPHA | 35 | "DMAA" | 13 | Heat | PP/GL ($17.4mN \cdot m^{-1}$) | E | E |
| | | Example 6 | 40 | HHPA | 2 | | | | | | |
| Comparative Examples | 21 | Comparative Example 5 | 97 | | | O-184 | 3 | UV | PET/PET ($0.0mN \cdot m^{-1}$) | F | P |
| | 22 | Comparative Example 2 | 50 | UV-7600B | 30 | IBOA | 15 | UV | PMMA/SST ($4.0mN \cdot m^{-1}$) | P | P |
| | | | | | | O-184 | 5 | | | | |
| | 23 | Comparative Example 4 | 50 | DPHA | 40 | O-184 | 10 | UV | PET/PET ($0.0mN \cdot m^{-2}$) | F | P |
| | 24 | Comparative Example 1 | 20 | UV-7600B | 60 | VS-1057 | 10 | UV | PMMA/SST ($4.0mN \cdot m^{-2}$) | P | P |
| | | | | | | TPO | 10 | | | | |

Examples 83 to 97 and Comparative Examples 25 to 28 (Production and evaluation of cosmetic composition)

Examples 83 to 89 and Comparative Examples 25 and 26 (Production and evaluation of hair cosmetic composition)

100 g of ethanol was added to a 1 L four-necked flask equipped with a reflux condenser, a thermometer, a nitrogen replacement tube, and a stirrer, and the product of polymerization obtained in Table 1, the polymerizable composition obtained in Table 2, and other components were further added in the proportion (in terms of solid content) shown in Table 9, and then a polymerization reaction was performed in a reflux state (about 80°C) for 8 hours under a

nitrogen stream. After completion of the polymerization reaction, 2-amino-2-methyl-1-propanol (AMP) (in terms of solid content) diluted with the same amount of ethanol was added at 50°C as a basic compound in the proportion shown in Table 9 for neutralization, and further diluted with ethanol so as to have a solid content of 40% to obtain a hair cosmetic base. The obtained hair cosmetic base, ethanol and liquefied petroleum gas were mixed at a weight ratio of 7.5 : 42.5 : 50 and enclosed in a spray can to obtain a hair spray type hair cosmetic. The moisture resistance, smoothness, anti-stickiness, texture, and stability over time of the obtained hair cosmetic were evaluated by the following method, and the results are shown in Table 9. Note that the polymerizable composition of Example 23 in Example 83 and the polymerizable composition of Example 28 in Example 84 were used without the polymerization initiator.

(Set retaining force (Moisture resistance) evaluation by curl retention method)

0.4 g of test formulation was sprayed onto a hair bundle having a length of 22 cm and a weight of 2 g and spread over the whole with a comb. The hair bundle was wound around a curler having a diameter of 2.2 cm and dried at a temperature of 20°C for 20 hours. The hair bundle was spirally unwound and attached to a vertically erected graduated glass plate, allowed to stand in a thermo-hygrostat controlled at 30°C and 95% RH, the tip position of the hair after a lapse of 10 hours was recorded, and the curl retention was calculated based on the following formula and evaluated according to the following criteria.

$$\text{Curl retention (\%)} = \{(L - Lt)/(L - L0)\} \times 100$$

L: length when test hair is stretched
Lt: tip position of test hair left in a thermo-hygrostat after a lapse of 10 hours
L0: tip position of test hair before putting in thermo-hygrostat
Excellent (E): 80% or more
Good (G): 65% or more and less than 80%
Fair (F): 50% or more and less than 65%
Poor (P): less than 50%

(Smoothness evaluation)

A dry hair bundle having a length of 22 cm and a weight of 2 g was sprayed with the test formulation, and immediately after spraying until drying (before drying) and after the drying, the ease of passing when the hair bundle was manually combed by an in-house tester was determined and evaluated according to the following criteria.

Excellent (E): no tack or creak before and after drying and slippage is good
Good (G): slippage is good before drying but slight tack and creak after drying
Fair (F): slight tack and creak before drying and tack and creak are strong after drying
Poor (P): tack and creak are strong before and after drying and impractical

(Stickiness resistance evaluation)

A dried hair bundle was prepared, sprayed with the test formulation, and the stickiness when the hair bundle after drying was gripped with a palm was evaluated.

Excellent (E): not sticky and smooth when touched with fingers
Good (G): slightly sticky when touched with fingers
Fair (F): sticky when touched with fingers
Poor (P): sticky and hard to be separated from fingers when touched with fingers

(Texture evaluation)

In the same manner as in the case of the moisture resistance evaluation, the feeling when the hair prepared was touched with a hand was evaluated as follows by a sensory test by the in-house tester, and further a change over time when the same test was performed after a lapse of one day was also evaluated. Evaluation criteria are shown below.

Excellent (E): smooth and dry touch
Good (G): slightly coarse but satisfactory

Fair (F): coarse or sticky
Poor (P): considerably coarse or strongly sticky

(Stability over time evaluation)

After the test formulation was allowed to stand at room temperature for one month, the degree of separation of formulation components was visually observed and evaluated as follows.

Excellent (E): no separation occurred at all
Good (G): slightly separated, shaken for 1 minute and allowed to stand for 7 days but no separation occurred
Fair (F): slightly separated, redispersible by shaking for 1 minute but separation occurred again after 1 hour
Poor (P): separation occurred, and could not be dispersed again even if shaken

Table 9

| | | Cosmetic composition | | Others | | Moisture resistance | Smoothness | Stickiness resistance | Texture | Stability over time |
|---|---|---|---|---|---|---|---|---|---|---|
| | | Type of product of polymerization or polymerizable composition | wt% | Type | wt% | | | | | |
| Examples | 83 | Example 3<br>Example 23 | 23<br>70 | 50HB-55<br>AIBN<br>AMP | 4<br>1<br>2 | E | E | G | G | E |
| Examples | 84 | Example 7<br>Example 28 | 10<br>82 | 50HB-55<br>AIBN<br>AMP<br>TBAA | 4<br>1<br>2<br>1 | E | E | G | E | E |
| Examples | 85 | Example 14 | 92 | 50HB-260<br>AIBN<br>AMP<br>TBAA | 4<br>1<br>2<br>1 | E | E | E | E | E |
| Examples | 86 | Example 15 | 93 | 50HB-100<br>AMP | 5<br>2 | E | E | G | G | G |
| Examples | 87 | Example 16 | 94 | 50HB-100<br>AMP | 4.5<br>1.5 | E | E | E | G | E |
| Examples | 88 | Example 18 | 92 | 50HB-260<br>AMP | 6<br>1 | G | G | G | G | G |
| Examples | 89 | Example 21 | 93 | 50HB-55<br>AMP | 5.5<br>1.5 | F | E | G | F | G |
| Comparative Examples | 25 | Comparative Example 4 | 92 | 50HB-55<br>AIBN<br>AMP | 4<br>2<br>2 | P | F | F | P | G |
| Comparative Examples | 26 | Comparative Example 5 | 93 | 50HB-55<br>AIBN<br>AMP | 4<br>1<br>2 | P | F | P | F | P |

Examples 90 to 97 and Comparative Examples 27 and 28 (Production and evaluation of skin cosmetic composition)

100 g of a water-ethanol mixed solvent (weight ratio 75:25) was added to a 1 L three-necked flask equipped with a reflux tube and a nitrogen inlet tube, and the product of polymerization obtained in Table 1, the polymerizable composition obtained in Table 2, and other components were added in the proportion (in terms of solid content) shown in Table 10. After the mixture was sufficiently dissolved or dispersed, it was purged with nitrogen for 20 minutes to remove dissolved oxygen. Thereafter, a polymerization reaction was performed by maintaining the temperature at 65 to 70°C for 8 hours in an oil bath while stirring. After completion of the polymerization, a dispersion liquid obtained by returning the polymerization liquid to room temperature was used as a cosmetic raw material. 1 g of a carboxyvinyl polymer as an aqueous phase component and 0.6 g of potassium hydroxide were added to ion-exchanged water and mixed, and added, stirred and mixed with 100 g of the cosmetic raw material separately dispersed in ion-exchanged

water. Note that the total amount of ion-exchanged water used was 500 g. After the cosmetic raw material and the aqueous phase component were uniformly dispersed, 100 parts of liquid paraffin, 100 g of glycerin tri-2-ethylhexanoate, and dimethylpolysiloxane (6 cs) were added as oil phase components, and sheared and mixed with a homomixer until the mixture became uniform to obtain an oil-in-water emulsified cosmetic. The emulsification stability, skin irritation, usability, and stability over time of the obtained oil-in-water emulsified cosmetic were evaluated by the following method, and the results are shown in Table 10. Note that the polymerizable composition of Example 13 in Example 90, the polymerizable composition of Example 17 in Example 91, the polymerizable composition of Example 19 in Example 95, and the polymerizable composition of Example 28 in Example 97 were used without the polymerization initiator.

(Emulsification stability (emulsified particles) evaluation)

Emulsified particles of a sample were observed with an optical microscope.

Excellent (E): emulsified particles were uniform, and no coalescence or aggregation was observed

Good (G): emulsified particles were almost uniform, and no coalescence or aggregation was observed

Fair (F): emulsified particles were almost uniform, but slight coalescence or aggregation was observed

Poor (P): emulsified particles were not uniform, and significant coalescence and aggregation were observed

(Skin irritation test)

Occlusive patches were applied to inner parts of upper arms of 10 sensitive skin panels for 24 hours, and the skin condition was determined according to the following criteria.

0: no abnormality is observed
1: slight redness is observed
2: redness is observed
3: redness and papules are observed

Evaluation criteria of "skin irritation test" are as follows.

Excellent (E): average value of 10 panels is 0 or more and less than 0.15
Good (G): average value of 10 panels is 0.15 or more and less than 0.2
Fair (F): average value of 10 panels is 0.2 or more and less than 0.3
Poor (P): average value of 10 panels is 0.3 or more

(Usability evaluation)

The usability ("anti-stickiness", "rich feeling", and "quick familiarity") when a sample was applied to the skin was evaluated by 10 expert panels according to the following criteria.

Excellent (E): 7 or more panels answered "good" or "can realize"
Good (G): 5 or more and less than 7 panels answered "good" or "can realize"
Fair (F): 3 or more and less than 5 panels answered "good" or "can realize"
Poor (P): 2 or less panels answered "good" or "can realize"

(Stability over time evaluation)

The state of the oil-in-water emulsified cosmetic 1 month after a lapse of the production was observed with the naked eye.

Excellent (E): sample is kept in an emulsified state at the time of production
Good (G): some settling or floating is observed, but the sample is almost kept in an emulsified state
Fair (F): emulsified particles settle or float, and coalescence of particles is also observed
Poor (P): emulsified particles in sample settle or float and coalesce, and oil phase is completely separated

Table 10

| | | Cosmetic composition | | Others | | Emulsification stability | Skin irritation | Usability | | | Stability over time |
|---|---|---|---|---|---|---|---|---|---|---|---|
| | | Type of product of polymerization or polymerizable composition | wt% | Type | wt% | | | Anti-stickiness | Rich feeling | Quick familiarity | |
| Examples | 90 | Example 2 | 33 | PME4000 | 6 | E | E | G | E | G | E |
| | | Example 13 | 60 | AIBN | 1 | | | | | | |
| | 91 | Example 7 | 7 | PME4000 | 6 | E | G | G | G | E | E |
| | | Example 17 | 85 | AIBN | 2 | | | | | | |
| | 92 | Example 14 | 92 | PME4000 | 7 | E | E | E | G | G | E |
| | | | | AIBN | 1 | | | | | | |
| | 93 | Example 16 | 93 | PME4000 | 5 | E | G | E | G | E | E |
| | | | | TBAA | 2 | | | | | | |
| | 94 | Example 18 | 90 | PME4000 | 7 | G | E | E | E | E | G |
| | | | | TBAA | 3 | | | | | | |
| | 95 | Example 15 | 14 | PME4000 | 5 | G | G | G | G | G | G |
| | | Example 19 | 80 | AIBN | 1 | | | | | | |
| | 96 | Example 21 | 3 | PME4000 | 7 | F | E | G | G | G | F |
| | 97 | Example 28 | 92.5 | PME4000 | 6 | E | G | G | G | G | E |
| | | | | AIBN | 0.5 | | | | | | |
| Comparative Examples | 27 | Comparative Example 6 | 93 | PME4000 | 6 | P | F | P | P | F | P |
| | | | | AIBN | 1 | | | | | | |
| | 28 | Comparative Example 7 | 93 | PME4000 | 6 | P | P | F | F | F | P |
| | | | | AIBN | 1 | | | | | | |

Examples 98 to 105 and Comparative Examples 29 and 30 (Preparation and evaluation of coating agent composition)

The product of polymerization obtained in Table 1, the polymerizable composition obtained in Table 2, and other components were weighed in the proportion shown in Table 11 (in terms of solid content), and uniformly mixed at room temperature to prepare a coating agent composition. Coating test pieces (coating films) were prepared by the following methods, and the wettability to various base materials (substrates) and the pencil hardness and adhesiveness of the coating films were evaluated, and the results are shown in Table 11.

(Preparation of coating test piece (coating film))

The coating agent composition was dropped in a strip shape onto a front end of various base materials (substrates), applied with a bar coater (RDS 3), and dried at 90°C for 2 minutes. Thereafter, curing was performed by a curing method (UV, EB, heat, UV heat, or EB heat) shown in Table 11 to form a coating layer on the base material (substrate). Subsequently, the film was placed in an environment of a temperature of 23°C and a relative humidity of 50% for 1 day to obtain a coating test piece for evaluation. Note that the UV curing method is a method in which the coating surface is directed upward and irradiated with ultraviolet rays (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 700 mW/cm$^2$, integrated light quantity: 1000 mJ/cm$^2$) to cure the film, and the EB curing is a method in which electron beams are irradiated instead of ultraviolet rays to cure the film. As an EB irradiation device, Curetron EBC-200-AA3 manufactured by Nisshin High Voltage Corporation was used (acceleration voltage: 200 kV, irradiation dose: 20 kGy). Further, the UV thermal curing is a method in which after UV curing, aging is further performed at 40°C for 72 hours to complete the crosslinking reaction by the crosslinking agent. Furthermore, the EB thermal curing is a method in which after EB curing, aging is further performed at 40°C for 72 hours to complete the crosslinking reaction by the crosslinking agent.

(Evaluation of wettability of coating agent composition)

The coating agent composition was applied to various substrates with a bar coater (RDS 3), and the degree of cissing of the coating liquid was visually observed.

Excellent (E): no cissing and coating film is uniform
Good (G): very little cissing but coating film is almost uniform
Fair (F): some cissing but coating film is almost uniform as a whole
Poor (P): many cissing and coating film is non-uniform

(Pencil hardness evaluation)

The evaluation was performed according to JIS K 5400 8.4, Hand scratching method (1990 edition).

(Adhesiveness evaluation)

According to JIS K 5600, 100 squares of 1 mm square were prepared, and the number of squares in which the adhesive layer remained on the substrate side when the cellophane tape was attached and peeled off at once was counted, and the adhesiveness was evaluated according to the following criteria.

Excellent (E): 100 pieces were not peeled off
Good (G): 95 to 99 pieces were not peeled off
Fair (F): 70 to 94 pieces were not peeled off
Poor (P): 0 to 69 pieces were not peeled off

Table 11

| | | Coating agent composition | | | | | | Curing method | Substrate | Wettability | Pencil hardness | Adhesiveness |
|---|---|---|---|---|---|---|---|---|---|---|---|---|
| | | Product of polymerization or polymerizable composition | | Crosslinking agent | | Others | | | | | | |
| | | Type | wt% | Type | wt% | Type | wt% | | | | | |
| Examples | 98 | Example 1 | 50 | PETA | 50 | | | UV | PET | E | 3H | E |
| | 99 | Example 3 | 50 | HDDA | 40 | "DMAA" | 10 | UV | PET | E | 2H | E |
| | 100 | Example 6 | 50 | DPHA | 30 | O-184 | 3 | UV | PMMA | E | 3H | E |
| | | | | "Quick Cure" 8100 | 17 | | | | | | | |
| | 101 | Example 5 | 50 | DPHA | 40 | "HEAA" | 5 | UV heat | PC | E | 3H | E |
| | | | | HDI | 3 | O-1173 | 2 | | | | | |
| | 102 | Example 8 | 25 | PETA | 25 | "HEAA" | 13 | EB heat | PP | E | 2H | E |
| | | Example 27 | 25 | HHPA | 2 | MHAGE | 10 | | | | | |
| | 103 | Example 17 | 50 | DPHA | 20 | "DEAA" | 15 | EB | PE | E | 2H | E |
| | | | | TPGDA | 15 | | | | | | | |
| | 104 | Example 12 | 20 | PETA | 30 | "HEAA" | 5 | UV heat | GL | E | 3H | G |
| | | Example 20 | 40 | HDI | 5 | | | | | | | |
| | 105 | Example 28 | 50 | DPHA | 35 | "DEAA" | 12 | UV | Al | E | 3H | G |
| | | | | | | KE-359 | 3 | | | | | |
| Comparative Examples | 29 | Comparative Example 3 | 50 | PETA | 45 | O-1173 | 5 | UV | PET | G | B | P |
| | 30 | Comparative Example 6 | 50 | DPHA | 20 | VS-1057 | 10 | UV | PMMA | P | H | P |
| | | | | UV-3000B | 15 | O-184 | 5 | | | | | |

Part by weight of adhesive composition is parts by weight of solid content after drying.

(Examples 106 to 113 and Comparative Examples 31 to 33)

The product of polymerization obtained in Table 1, the polymerizable composition obtained in Table 2, and other components were weighed in the proportion shown in Table 12 (in terms of solid content), and uniformly mixed at room temperature to prepare a ink composition. The viscosity of the prepared ink composition was measured to evaluate the pigment dispersibility. Further, using the prepared ink composition, inkjet printing was performed, and physical

properties of the obtained printed matter were evaluated. Note that in a clear ink composition, the pigment and the pigment dispersant were not blended, and the black ink composition was blended with the pigment Pigment Black 7, and evaluations were made for each of those containing and not containing the pigment dispersant AJISPER PB821. The evaluation results are summarized in Table 12.

(Viscosity measurement and evaluation)

The viscosity of the ink composition was measured by a cone plate viscometer (RE550 type viscometer manufactured by Toki Sangyo Co., Ltd.) according to JIS K5600-2-3. As an ink composition for inkjet printing, the viscosity was evaluated in four stages as follows.

Excellent (E): 5 to less than 100 mPa·s
Good (G): 100 to less than 500 mPa·s
Fair (F): 500 to less than 2000 mPa·s
Poor (P): 2000 mPa s or more

(Pigment dispersibility evaluation)

Using the prepared ink composition, the aggregation and precipitation state of the pigment immediately after the preparation and after standing for 2 months were visually observed, and the pigment dispersibility was evaluated in four stages as follows.

Excellent (E): no aggregation and precipitation of pigment was observed immediately after preparation and after standing for 2 months
Good (G): no precipitation of pigment was observed immediately after preparation, but was slightly observed after standing for 2 months
Fair (F): aggregation or precipitation of pigment was slightly observed immediately after preparation, but was clearly observed after standing for 2 months
Poor (P): aggregation and precipitation of pigment were clearly observed even immediately after the preparation

Method for preparing printed matter by ultraviolet irradiation

The obtained ink composition was applied to a PET film having a thickness of 100 μm with a bar coater (RDS 12) (film thickness after drying: 10 μm), and cured by ultraviolet irradiation (Inverter type conveyor device ECS-4011GX and metal halide lamp M04-L41 manufactured by Eye Graphics Co., Ltd.) to prepare a printed matter.

(Curability evaluation)

When the printed matter was produced by the above method, the integrated light amount until the ink composition was completely cured (non-sticky state) was measured, and the curability was evaluated.

Excellent (E): completely cured at 1000 $mJ/cm^2$
Good (G): completely cured at 1000 to 2000 $mJ/cm^2$
Fair (F): completely cured at 2000 to 5000 $mJ/cm^2$
Poor (P): 5000 $mJ/cm^2$ or more is required until complete curing

(Surface dryness evaluation)

The printed matter prepared by the above method was allowed to stand in an environment of a room temperature of 23°C and a relative humidity of 50% for 5 minutes, a high-quality paper was overlaid on the printed surface, a load of 1 $kg/cm^2$ was applied for 1 minute, and the degree of transfer of the ink to the paper was evaluated.

Excellent (E): ink was dried and not transferred to paper at all
Good (G): ink was dried and slightly transferred to paper
Fair (F): ink was almost dried and transferred to paper
Poor (P): ink was dried very little and much transferred to paper

(Adhesiveness evaluation)

The obtained ink composition was applied to various substrates, and irradiated with ultraviolet rays (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 700 $mW/cm^2$, integrated light quantity: 1000 $mJ/cm^2$) to be completely cured. 100 squares of 1 mm square were prepared from the obtained cured film by a cross-cut test method, and the number of squares in which the cured film remained on the substrate side when the cellophane tape was attached and peeled off at once was counted and evaluated.

Excellent (E): the number of remaining squares is 100
Good (G): the number of remaining squares is 90 or more and less than 100
Fair (F): the number of remaining squares is 50 or more and less than 90
Poor (P): the number of remaining squares is less than 50

(Inkjet printing and printability evaluation)

The ink composition prepared above was filled in a commercially available inkjet printer (LuxelJet UV350GTW manufactured by FUJIFILM Corporation), a solid image was printed using coated paper, and the printability of the ink was evaluated by the following method.

(Discharge stability evaluation)

The printing state of the obtained printed matter was visually evaluated.

Excellent (E): good printing without missing nozzles
Good (G): slight missing nozzles
Fair (F): missing nozzles in a wide range
Poor (P): non-discharge

(Sharpness evaluation)

Image sharpness of the printed matter obtained from the ink composition containing a pigment was visually observed.

Excellent (E): ink bleeding was not observed at all, and the image was clear
Good (G): ink bleeding was almost not observed, and the image was good
Fair (F): ink bleeding was slightly observed
Poor (P): ink bleeding was significantly observed

(Yellowing resistance evaluation)

The resulting clear ink composition was applied to a substrate (#125-E20) with a bar coater (RDS 12) so as to have a film thickness of 10 $\mu$m, and cured with a metal halide lamp in the same manner as described above. The hue of the obtained coating film was measured by Spcetrolino (manufactured by GretagMacbeth), and the coating film was allowed to stand in a thermostat maintained at 60°C for 1 week. Thereafter, the hue of the coating film was measured again, and the yellowing resistance was evaluated by the hue value change before and after heating ($\Delta E$ = hue after heating - hue before heating).

Excellent (E): $0 \leq \Delta E \leq 0.2$
Good (G): $0.2 < \Delta E \leq 0.5$
Fair (F): $0.5 < \Delta E \leq 1.0$
Poor (P): $1.0 < \Delta E$

Table 12

| | | Ink composition | | | | | | Viscosity | Pigment dispersibility | Curability | Surface dryness | Adhesiveness | | | Ejection stability | Sharpness | Heat yellowing resistance |
|---|---|---|---|---|---|---|---|---|---|---|---|---|---|---|---|---|---|
| | | Product of polymerization or polymerizable composition | | Crosslinking agent | | Others | | | | | | | | | | | |
| | | Type | wt (%) | Type | wt (%) | Type | wt (%) | | | | | PE | PP | PET | | | |
| Examples | 106 | Example 1 | 85 | TPGDA | 15 | | | E | - | E | E | E | E | E | E | - | E |
| | 107 | Example 2 | 72 | PETA | 20 | Black pigment | 3 | E | E | E | E | E | E | E | E | E | - |
| | | | | | | Pigment dispersant | 2 | | | | | | | | | | |
| | | | | | | TPO | 3 | | | | | | | | | | |
| | 108 | Example 20 | 50 | | | "DMAA" | 40 | E | E | E | G | G | E | E | E | E | - |
| | | | | | | Black pigment | 3 | | | | | | | | | | |
| | | | | | | O-184 | 7 | | | | | | | | | | |
| | 109 | Example 23 | 70 | | | IBOA | 30 | E | - | E | E | E | E | E | E | - | E |
| | 110 | Example 19 | 20 | HDDA | 30 | Black pigment | 3 | E | G | E | E | E | E | E | E | G | - |
| | | | | | | Pigment dispersant | 2 | | | | | | | | | | |
| | | Example 5 | 20 | TPGDA | 20 | TPO | 5 | | | | | | | | | | |
| | 111 | Example 22 | 90 | PETA | 10 | | | E | - | E | E | E | E | E | E | - | E |
| | 112 | Example 26 | 5 | UV-6600B | 55 | DAAM | 30 | E | E | G | E | G | E | E | E | E | - |
| | | | | | | Black pigment | 3 | | | | | | | | | | |
| | | Example 2 | 5 | | | TPO | 2 | | | | | | | | | | |
| | 113 | Example 28 | 60 | "Quick Cure" 8100 | 10 | IBOA | 20 | E | G | E | G | E | E | E | E | E | E |
| | | | | | | Black pigment | 3 | | | | | | | | | | |
| | | | | | | Pigment dispersant | 2 | | | | | | | | | | |
| | | | | | | TPO | 5 | | | | | | | | | | |
| Comparative Examples | 31 | Comparative Example 2 | 30 | TPGDA | 30 | PEA | 25 | G | - | F | P | P | P | F | P | - | P |
| | | | | | | IBOA | 10 | | | | | | | | | | |
| | | | | | | O-184 | 5 | | | | | | | | | | |
| | 32 | Comparative Example 5 | 40 | PETA | 30 | IBOA | 20 | F | F | P | P | P | P | P | F | P | - |
| | | | | | | Black pigment | 3 | | | | | | | | | | |
| | | | | | | Pigment dispersant | 2 | | | | | | | | | | |
| | | | | | | TPO | 5 | | | | | | | | | | |
| | 33 | Comparative Example 7 | 70 | DPHA | 20 | Black pigment | 3 | F | P | P | P | F | P | P | P | F | - |
| | | | | | | TPO | 7 | | | | | | | | | | |

(Examples 114 to 122 and Comparative Examples 34 and 35)

The product of polymerization obtained in Table 1, the polymerizable composition obtained in Table 2, and other components were weighed in the proportion shown in Table 13 (in terms of solid content), and uniformly mixed at room temperature to prepare a three-dimensional modeling ink composition. The curing shrinkage rate of the three-dimensional modeling ink composition and the strength, heat resistance, water resistance, and modeling accuracy of the cured product of the three-dimensional modeling ink composition were measured by the following methods. The evaluation results are shown in Table 13.

(Curing shrinkage resistance evaluation)

The curing shrinkage rate was determined by the density change before and after curing of the three-dimensional modeling ink composition as shown in the following calculation formula (1) according to JIS K5600-2-4. The density before and after curing of the three-dimensional modeling ink composition was measured by an electronic densimeter (MDS-300 manufactured by Alfa Mirage Co., Ltd.) according to JIS K7112. The cured product was prepared in the same manner as the test piece for the tensile test. The curing shrinkage rate was evaluated from the obtained curing shrinkage rate as follows.

$$\text{(Curing shrinkage rate)} = (Ds - Dl)/Dl \times 100 \cdots \text{Calculation formula (1)}$$

(where Ds is the density after curing of the three-dimensional modeling ink composition, and Dl is the density before curing of the three-dimensional modeling ink composition)

Excellent (E): curing shrinkage rate is less than 6%
Good (G): curing shrinkage rate is 6% or more and less than 7%
Fair (F): curing shrinkage rate is 7% or more and less than 8%
Poor (P): curing shrinkage rate is 8% or more

(Strength evaluation)

A heavy release PET film (polyester film E7001 manufactured by Toyobo Co., Ltd.) having a thickness of 75 μm was brought into close contact with a horizontally placed glass plate, a spacer having a thickness of 1 mm and an inside punched into a No. 2 dumbbell type according to JIS K6251 was placed, the inside of the spacer was filled with the three-dimensional modeling ink composition obtained in each of Examples and Comparative Examples, then a light release PET film (polyester film E7002 manufactured by Toyobo Co., Ltd.) having a thickness of 50 μm was further overlaid thereon, and both surfaces thereof were irradiated with ultraviolet rays (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 200 mW/cm$^2$, integrated light quantity: 1000 mJ/cm$^2$) to cure the three-dimensional modeling ink composition. Thereafter, the release PET film on both sides was removed to obtain test pieces of the cured products for Examples and the cured products for Comparative Examples. The tensile strength was measured under conditions of a tensile speed of 10 mm/min and a distance between chucks of 50 mm in a temperature environment of 25°C using a desktop precision universal tester (autograph AGS-X manufactured by Shimadzu Corporation) according to JIS K7161, and the strength was evaluated according to the following criteria.

Excellent (E): tensile strength is 40MPa or more
Good (G): tensile strength is 30MPa or more and less than 40MPa
Fair (F): tensile strength is 20MPa or more and less than 30MPa
Poor (P): tensile strength is less than 20MPa

(Heat resistance evaluation)

A cured product was prepared in the same manner as the test piece for the tensile test, and the glass transition temperature (Tg) of the cured product was measured by a differential scanning calorimeter (DSC-60 plus manufactured by Shimadzu Corporation). The heat resistance was evaluated as follows from the measured value of the glass transition temperature (Tg) of the cured product.

Excellent (E): Tg of cured product is 80°C or higher
Good (G): Tg of cured product is 40°C or higher and lower than 80°C
Poor (P): Tg of cured product is lower than 40°C

(Water resistance evaluation)

A heavy release PET film (polyester film E7001 manufactured by Toyobo Co., Ltd.) having a thickness of 75 μm was brought into close contact with a horizontally placed glass plate, a spacer having a thickness of 10 mm and an inside of 10 cm × 1 cm was placed, the inside of the spacer was filled with the three-dimensional modeling ink composition having a thickness of 1 mm obtained in each of Examples and Comparative Examples, the surface was smoothed by

incubating at 60°C for 30 seconds, and then the three-dimensional modeling ink composition was cured by being irradiated with ultraviolet rays (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 200 mW/cm$^2$) to obtain a cured product having a length of 10 cm, a width of 1 cm, a thickness of 1 mm. The weight of the obtained cured product immediately after production was measured, then the cured product was immersed in a beaker containing 100 ml of water, and the weight after immersion was measured one day later. The water absorption was measured by substituting the weight before immersion and the weight after immersion into the following formula, and the water resistance was evaluated according to the following criteria.

Excellent (E): water absorption is less than 2%
Good (G): water absorption is 2% or more and less than 2.5%
Fair (F): water absorption is 2.5% or more and less than 3%
Poor (P): water absorption is 3% or more

(Modeling accuracy evaluation)

A heavy release PET film (polyester film E7001 manufactured by Toyobo Co., Ltd.) having a thickness of 75 μm was brought into close contact with a horizontally placed glass plate, a spacer having a thickness of 10 mm and an inside of 10 × 10 mm was placed, the inside of the spacer was filled with the three-dimensional modeling ink composition having a thickness of 1 mm obtained in each of Examples and Comparative Examples, the surface was smoothed by incubating at 60°C for 30 seconds, and then the three-dimensional modeling ink composition was cured by being irradiated with ultraviolet rays (apparatus: inverter type conveyor apparatus ECS-4011GX manufactured by Eye Graphics Co., Ltd., metal halide lamp: M04-L41 manufactured by Eye Graphics Co., Ltd., ultraviolet illuminance: 200 mW/cm$^2$). Thereafter, the three-dimensional modeling ink composition was filled in a thickness of 1 mm, and curing was repeated 10 times in total to obtain a cured product of 10 × 10 × 10 mm. The height of the obtained cured product was measured. In addition, the side surface of the obtained cured product was visually observed. These results were combined, and the modeling accuracy was evaluated according to the following criteria.

Excellent (E): height is less than 10 mm ± 0.1 mm, and no irregularities on side surface
Good (G): height is 10 mm ± 0.1 mm or more and less than ± 0.2 mm, or very slight irregularities on side surface
Fair (F): height is 10 mm ± 0.2 mm or more and less than ± 0.3 mm, or slight irregularities on side surface
Poor (P): height is 10 mm ± 0.3 mm or more, or obvious irregularities on side surface

Table 13

| | | Three-dimensional modeling ink composition | | | | | | Rubber shore hardness D | Elongation at break (%) | Tensile strength (MPa) |
|---|---|---|---|---|---|---|---|---|---|---|
| | | Product of polymerization or polymerizable composition | | Crosslinking agent | | Others | | | | |
| | | Type | wt% | Type | wt% | Type | wt% | | | |
| Examples | 114 | Example 1 | 10 | HDDA | 30 | "DMAA" | 55 | 70 | 21 | 15 |
| | | | | | | TPO | 5 | | | |
| | 115 | Example 5 | 4 | PETA | 20 | "DMAA" | 25 | 73 | 17 | 19 |
| | | Example 26 | 50 | | | TPO | 1 | | | |
| | 116 | Example 17 | 10 | DPHA | 50 | "DEAA" | 30 | 76 | 23 | 25 |
| | | | | "Quick Cure" 8100 | 10 | | | | | |
| | 117 | Example 20 | 80 | HDDA | 10 | | | 71 | 15 | 21 |
| | | | | TPGDA | 10 | | | | | |
| | 118 | Example 28 | 70 | PETA | 30 | | | 70 | 24 | 16 |
| Comparative Examples | 34 | Comparative Example 2 | 20 | TPGDA | 50 | "DMAA" | 20 | 51 | 12 | 8 |
| | | | | | | O-1173 | 10 | | | |
| | 35 | Comparative Example 7 | 50 | DPHA | 30 | O-184 | 5 | 63 | 7 | 13 |
| | | | | UV-6640 | 15 | | | | | |

As shown in the evaluation results of Examples and Comparative Examples described above, it is found that the polymerizable composition containing N-substituted (meth)acrylamide (A) having a specific structure according to the present invention has high transparency and good curability, and is excellent in wettability to the substrates of various materials having a wide range of polarities from low to high polarity, such as the substrate of organic material, the substrate of inorganic material, and the substrate of the organic-inorganic hybrid material, due to well-balanced amphiphilicity of A, and the product of polymerization or the cured product of the polymerizable composition is excellent in water resistance due to the influence of the hydrophobic substituent of A. An adhesive composition containing the polymerizable composition and/or the product of polymerization of the polymerizable composition, and the like, and a multi-layer laminate product of an adhesive layer formed of the adhesive composition and various substrates exhibit adhesiveness and adhesive strength to various substrates, and have high transparency, stain resistance, yellowing resistance and durability. The glue composition containing the polymerizable composition and/or the product of polymerization of the polymerizable composition, and the like has high bond strength to various substrates, impact resistance, and water resistance, and can be used as a glue composition for the same or different types of materials. The cosmetic composition containing the polymerizable composition and/or the product of polymerization of the polymerizable composition, and the like can be used as a hair cosmetic having moisture resistance, smoothness, anti-stickiness, good texture, and stability over time, or an oil-in-water emulsified cosmetic composition that does not exhibit skin irritation, and has excellent emulsification stability, usability, and stability over time. It is apparent that the coating agent composition containing the polymerizable composition and/or the product of polymerization of the polymerizable composition, and the like has high wettability and adhesiveness to various substrates, and exhibits high surface hardness and water resistance by being cured, and the ink composition has high adhesiveness to various substrates, is excellent in printing characteristics such as pigment dispersibility, surface dryness, ejection stability, and sharpness, and has high curability and yellowing resistance. Further, the three-dimensional modeling ink composition containing the polymerizable composition and/or the product of polymerization of the polymerizable composition, and the like can accurately form the three-dimensional shaped article having high strength, heat resistance, and water resistance. On the other hand, it is apparent that various molded articles obtained using the polymerizable composition not containing the N-substituted (meth)acrylamide (A) and/or the product of polymerization of the polymerizable composition, such as an adhesive composition, a glue composition, a cosmetic composition, a coating agent composition, an ink composition, and a three-dimensional modeling ink composition, are inferior in the effects of various molded articles obtained from the polymerizable composition containing A and/or the product of polymerization of the polymerizable composition described above.

## INDUSTRIAL APPLICABILITY

As described above, by containing a specific N-substituted (meth)acrylamide (A), the polymerizable composition of the present invention is excellent in wettability to various substrates having a wide range of polarities from low to high polarity while having high transparency and good curability, and the product of polymerization of the polymerizable composition is excellent in water resistance. Therefore, the polymerizable composition and/or the product of polymerization of the polymerizable composition can be used as an adhesive composition that can be polymerized and cured by active energy rays and/or heat, for adhesives and adhesive-related products in a wide range of fields such as industrial use, medical use, and home use. The adhesive composition has good adhesiveness to various substrates exhibiting a wide range of polarities, and for example, a polyolefin adhesive sheet and a polyimide adhesive sheet are obtained by forming an adhesive layer on a film-like or sheet-like substrate of resins such as a polyolefin such as polyethylene or polypropylene, a polycarbonate, an acrylonitrile-butadiene-styrene copolymer, a polyimide, or a polymethyl methacrylate. Further, when the adhesive layer is formed on glass or a metal substrate, a glass adhesive sheet and a metal adhesive sheet are obtained. Furthermore, it is possible to easily obtain an adhesive sheet for an electronic material including a substrate used for an electronic device and an adhesive layer, an adhesive sheet for an optical member including a substrate used for an optical member and an adhesive layer, and an adhesive sheet for an automobile including a substrate used for an automobile and an adhesive layer. The glue composition including the adhesive composition of the present invention and a crosslinking agent is very effective for bonding the same type of materials and bonding various different types of materials from plastics to metals, and can be widely used for electronic materials, optical components, semiconductors, solar cells, and the like. Further, the present invention can be used for a cosmetic composition excellent in moisture resistance, emulsification stability, and the like, and having good usability, a coating agent composition capable of providing a coating layer excellent in adhesiveness to various substrates and having high surface hardness and water resistance, an ink having high adhesiveness to various substrates, excellent in printing characteristics such as pigment dispersibility, surface dryness, ejection stability, and sharpness, and having high curability and yellowing resistance, and a three-dimensional modeling ink composition excellent in curing shrinkage resistance capable of accurately forming a three-dimensional shaped article having high strength, heat resistance, and water resistance.

**Claims**

1. A polymerizable composition comprising N-substituted (meth)acrylamide (A) represented by a general formula [1].

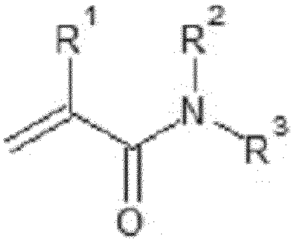

General Formula [1]

(In the formula, $R^1$ represents a hydrogen atom or a methyl group, one of $R^2$ and $R^3$ represents a chain hydrocarbon group having 6 or more carbon atoms or a cyclic hydrocarbon group having 6 or more carbon atoms, the other represents a hydrogen atom, a chain hydrocarbon group having 1 or more carbon atoms, or a cyclic hydrocarbon group having 3 or more carbon atoms, and $R^2$ and $R^3$ include a group in which a 6- or more-membered saturated ring is formed together with a nitrogen atom carrying $R^2$ and $R^3$.)

2. The polymerizable composition according to claim 1, wherein the N-substituted (meth)acrylamide (A) is N-mono-substituted (meth)acrylamide and N,N-disubstituted (meth)acrylamide, and has as substituents one or more structures selected from chain saturated and unsaturated structures and cyclic saturated and unsaturated structures having 6 or more and 36 or less carbon atoms.

3. The polymerizable composition according to claim 1 or 2, wherein a saturated water absorption of a cured product is 10% or less.

4. The polymerizable composition according to any one of claims 1 to 3, wherein the N-substituted (meth)acrylamide (A) has a surface tension of 24.0 to 46.0 $mN \cdot m^{-1}$.

5. The polymerizable composition according to any one of claims 1 to 4, wherein a content of the N-substituted (meth)acrylamide (A) is 1 wt% or more based on the entire polymerizable composition.

6. A product of polymerization obtained by polymerizing the polymerizable composition according to any one of claims 1 to 5 with active energy rays and/or heat.

7. The polymerizable composition according to any one of claims 1 to 5, further comprising one or more selected from a polymerization initiator, a compound having an unsaturated bond (excluding N-substituted (meth)acrylamide (A) and a product of polymerization using N-substituted (meth)acrylamide (A)), a non-polymerizable oligomer and a non-polymerizable polymer (excluding the product of polymerization using N-substituted (meth)acrylamide (A)), and the product of polymerization according to claim 6.

8. An adhesive composition comprising the polymerizable composition according to any one of claims 1 to 5 and 7 or the product of polymerization according to claim 6, or the polymerizable composition according to any one of claims 1 to 5 and 7 or the product of polymerization according to claim 6 and a crosslinking agent.

9. A multi-layer laminate product comprising an adhesive layer containing the adhesive composition according to claim 8 and an organic and/or inorganic substrate, wherein the organic and/or inorganic substrate has a surface tension of 22.6 to 59.0 $mN \cdot m^{-1}$.

10. A glue composition comprising the polymerizable composition according to any one of claims 1 to 5 and 7 or the product of polymerization according to claim 6 and a crosslinking agent.

11. A glue composition comprising the polymerizable composition according to any one of claims 1 to 5 and 7 or the product of polymerization according to claim 6 and a crosslinking agent, wherein an absolute value of a difference

in surface tension between two kinds of different types of adherends is 37.0 mN·$m^{-1}$ or less.

**12.** A cosmetic composition comprising the polymerizable composition according to any one of claims 1 to 5 and 7 or the product of polymerization according to claim 6.

**13.** A coating agent composition comprising the polymerizable composition according to any one of claims 1 to 5 and 7 or the product of polymerization according to claim 6, or the polymerizable composition according to any one of claims 1 to 5 and 7 or the product of polymerization according to claim 6 and a crosslinking agent.

**14.** An ink composition comprising the polymerizable composition according to any one of claims 1 to 5 and 7 or the product of polymerization according to claim 6, or the polymerizable composition according to any one of claims 1 to 5 and 7 or the product of polymerization according to claim 6 and a crosslinking agent.

**15.** An ink composition for use in three-dimensional modeling, comprising the polymerizable composition according to any one of claims 1 to 5 and 7 or the product of polymerization according to claim 6, or the polymerizable composition according to any one of claims 1 to 5 and 7 or the product of polymerization according to claim 6 and a crosslinking agent.

**INTERNATIONAL SEARCH REPORT**

International application No.
**PCT/JP2021/038313**

**A. CLASSIFICATION OF SUBJECT MATTER**

***C08F 20/56***(2006.01)i; ***C09J 4/02***(2006.01)i; ***C09J 7/38***(2018.01)i
FI: C08F20/56; C09J7/38; C09J4/02

According to International Patent Classification (IPC) or to both national classification and IPC

**B. FIELDS SEARCHED**

Minimum documentation searched (classification system followed by classification symbols)

C08F20/56; C09J4/02; C09J7/38

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 8-053520 A (MITSUBISHI CHEM CORP) 27 February 1996 (1996-02-27)<br>claims, paragraphs [0008]-[0014], [0022], examples | 1-2, 4-7 |
| X | WO 2018/070079 A1 (NIPPON SODA CO) 19 April 2018 (2018-04-19)<br>claims, paragraphs [0011]-[0030], [0036]-[0038], [0042]-[0049], [0057]-[0058], [0085]-[0086], [0102], [0107]-[0188] | 1, 3-7, 10-11, 13 |
| X | JP 2-180911 A (KAO CORP) 13 July 1990 (1990-07-13)<br>claims, page 4, upper left column, lines 2-14, page 4, lower left column, line 13 to page 5, upper right column, line 8, Effect of the invention, examples | 1, 4-7, 12-13 |
| P, X | JP 2020-200437 A (CANON KK) 17 December 2020 (2020-12-17)<br>claims, paragraphs [0031], [0064], [0081]-[0109] | 1, 4-7, 14-15 |
| X | JP 2003-513122 A (3M INNOVATIVE PROPERTIES COMPANY) 08 April 2003 (2003-04-08)<br>claims, paragraphs [0014], [0020], [0024], [0026], [0094]-[0102], [0116]-[0119], [0156]-[0197] | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 December 2021** | **28 December 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2021/038313**

**C. DOCUMENTS CONSIDERED TO BE RELEVANT**

| Category* | Citation of document, with indication, where appropriate, of the relevant passages | Relevant to claim No. |
|---|---|---|
| X | JP 7-157669 A (NIPPON SHOKUBAI CO LTD) 20 June 1995 (1995-06-20) claims, paragraphs [0041]-[0045] | 1, 3-7 |

Form PCT/ISA/210 (second sheet) (January 2015)

**INTERNATIONAL SEARCH REPORT**

International application No. **PCT/JP2021/038313**

**Box No. III Observations where unity of invention is lacking (Continuation of item 3 of first sheet)**

This International Searching Authority found multiple inventions in this international application, as follows:

(Invention 1) Claims 1-2 and claims 3-15 referring to claim 2
The invention in claim 1 cannot be said to have a special technical feature in light of the disclosure of document 1. Next, a special technical feature was found in claim 2. Therefore, the invention in claims 1-2 having the found special technical feature and claims 3-15 referring to claim 2 is classified as invention 1.
(Invention 2) Claims 3-7 which do not refer to claim 2
The invention in claims 3-7, which do not refer to claim 2, is considered to be inventively related, and is thus classified as invention 2.
(Invention 3) Claims 8-11 which do not refer to claim 2
The invention in claims 8-11 shares, with inventions 1 and 2, the common technical feature of a "polymerizable composition containing N-substituted (meth)acrylamide (A) represented by general formula [1]". However, said technical feature does not make a contribution over the prior art in light of the disclosure of document 1, and thus cannot be said to be a special technical feature. Moreover, there are no other same or corresponding special technical features between the invention in claims 8-11 and inventions 1 and 2. Furthermore, document 1 indicates that a polymer of a polymerizable composition satisfying the matters specified in claim 1 of the present application is also water-soluble, and as indicated in document 1, it cannot be said that all of the substances satisfying the matters specified in claim 1 of the present application have excellent water resistance. Consequently, it cannot be said that the invention in claim 1 of the present application and the invention in claims 8-11, which do not refer to claim 2 and have excellent water resistance as one of the problems, have relevance with respect to the problem. In addition, the invention classified as invention 1 or 2 and the invention in claims 8-11, which do not refer to claim 2, are not substantially identical to or similarly closely related to each other. Therefore, the invention in claims 8-11, which do not refer to claim 2, is classified as invention 3.
(Invention 4) Claim 12 which does not refer to claim 2
The invention in claim 12 has a common technical feature in terms of the matters specified in the invention in claim 1. However, said technical feature does not make a contribution over the prior art in light of the disclosure of document 1, and thus cannot be said to be a special technical feature. Moreover, there are no other same or corresponding special technical features between the invention in claim 12 and inventions 1-3. Furthermore, it cannot be said that the invention in claim 1 of the present application and the invention in claim 12, which does not refer to claim 2 and has high water resistance as one of the problems, have relevance with respect to the problem. In addition, the invention classified as any of inventions 1-3 and the invention in claim 12, which does not refer to claim 2, are not substantially identical to or similarly closely related to each other. Therefore, the invention in claim 12, which does not refer to claim 2, is classified as invention 4.
(Invention 5) Claim 13 which does not refer to claim 2
The invention in claim 13 has a common technical feature in terms of the matters specified in the invention in claim 1. However, said technical feature does not make a contribution over the prior art in light of the disclosure of document 1, and thus cannot be said to be a special technical feature. Moreover, there are no other same or corresponding special technical features between the invention in claim 13 and inventions 1-4. Furthermore, it cannot be said that the invention in claim 1 of the present application and the invention in claim 13, which does not refer to claim 2 and has humidity resistance as one of the problems, have relevance with respect to the problem. In addition, the invention classified as any of inventions 1-4 and the invention in claim 13, which does not refer to claim 2, are not substantially identical to or similarly closely related to each other. Therefore, the invention in claim 13, which does not refer to claim 2, is classified as invention 5.
(Invention 6) Claims 14-15 which do not refer to claim 2
The invention in claims 14-15 has a common technical feature in terms of the matters specified in the invention in claim 1. However, said technical feature does not make a contribution over the prior art in light of the disclosure of document 1, and thus cannot be said to be a special technical feature. Moreover, there are no other same or corresponding special technical features between the invention in claims 14-15 and inventions 1-5. Furthermore, it cannot be said that the invention in claim 1 of the present application and the invention in claims 14-15, which do not refer to claim 2 and have high water resistance as one of the problems, have relevance with respect to the problem. In addition, the invention classified as any of inventions 1-5 and the invention in claims 14-15, which do not refer to claim 2, are not substantially identical to or similarly closely related to each other. Therefore, the invention in claims 14-15, which do not refer to claim 2, is classified as invention 6.

Form PCT/ISA/210 (continuation of first sheet) (January 2015)

INTERNATIONAL SEARCH REPORT

International application No.
**PCT/JP2021/038313**

**Box No. III Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2021/038313**

| Patent document cited in search report | Publication date (day/month/year) | Patent family member(s) | Publication date (day/month/year) |
|---|---|---|---|
| JP 8-053520 A | 27 February 1996 | (Family: none) | |
| WO 2018/070079 A1 | 19 April 2018 | EP 3527642 A1<br>claims, paragraphs [0008]-[0013], [0016]-[0017], [0019]-[0026], [0034], [0057], [0067], [0070]-[0154]<br>CN 109804033 A | |
| JP 2-180911 A | 13 July 1990 | US 5278269 A<br>claims, column 3, lines 19-30, column 3, line 64 to column 4, line 40, column 6, lines 5-37, examples<br>EP 372546 A2 | |
| JP 2020-200437 A | 17 December 2020 | (Family: none) | |
| JP 2003-513122 A | 08 April 2003 | EP 1244719 B1<br>claims, paragraphs [0014], [0020], [0024], [0026], [0095]-[0103], [0117]-[0120], [0155]-[0189]<br>WO 2001/030872 A1 | |
| JP 7-157669 A | 20 June 1995 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008287207 A
- JP 2011122013 A
- JP 2001310918 A
- JP 2010155889 A
- JP 2011137181 A
- JP 2010235646 A
- JP 2013256552 A


### Non-patent literature cited in the description


- Chemical Engineering Handbook Revision. vol. 7, 66